# EUROPEAN PATENT APPLICATION

(11) **EP 4 736 935 A2**
(43) Date of publication of application: **06.05.2026**
(21) Application number: 26159379.2
(22) Date of filing: 10.12.2021
(51) Int. Cl.: A61N 1/36

(54) **DEVICE FOR INSERTION OF MICROFILAMENTS IN SOFT TISSUE**

(30) Priority: 10.12.2020 SE 2030357
(62) Divisional of application: 21903954.2
(71) Applicant: Neuronano AB, 37435 Karlshamn (SE)
(72) Inventor: SCHOUENBORG, Jens, 22353 Lund (SE)
(74) Representative: Rigler, Johann

(57) **Abstract**

The present invention relates to an implantable microfilament guiding structure comprising spatially arranged channels intended for the accommodation and movement of microfilaments such as electrically conductive microelectrodes. The channels of the microfilament guiding structure are spatially arranged such that the distances between at least some of said channels gradually increase in distal direction. The microfilament guiding structure comprising microfilaments forms part of a proto electrode also referred to as prior microfilament assembly. The invention also encompasses methods for manufacturing the microfilament guiding structure and proto microelectrode assemblies and further a method for implantation of microfilaments in soft tissue.

## Description

### FIELD OF INVENTION

The present invention relates to an implantable microfilament guiding structure comprising spatially arranged channels intended for the accommodation and movement of microfilaments such as electrically conductive microelectrodes. The microfilament guiding structure comprises materials which disintegrates and/or dissolves when subjected to mammalian tissue fluids. The channels of the microfilament guiding structure are spatially arranged such that the distances between the majority of said channels gradually increase in distal direction. The microfilament guiding structure comprising microfilaments forms part of a proto electrode also referred to as prior microfilament assembly. The invention also encompasses methods for manufacturing the microfilament guiding structure and proto microelectrode assemblies and further a method for implantation of microfilaments in soft tissue

### BACKGROUND OF THE INVENTION

Electrodes for implantation into the nervous system are widely used for recording neuronal electrical and chemo-electrical signals and may also be used for electrical stimulation of the nervous system at specific regions thereby ameliorating for example motor symptoms in Parkinson's disease or chronic pain.

The functionality of implantable electrodes depends on the injury caused by the electrodes on insertion, the severity of the tissue responses triggered by the implant and the extent of the loss of neurons nearby the implanted electrode. The tissue responses comprise glial reactions (e.g. such as activation of microglia and proliferation of astrocytes) that after a while may encapsulate the implant, thereby isolating the electrode(s) from the rest of the brain. As a consequence, the ability of the electrode(s) to record neuron signals often deteriorate with time. While size of the electrodes will impact on the initial injuries caused by the insertion, one important trigger of the glial reactions is microforces between implanted electrode and nearby tissue occurring when electrodes are unable to follow movements of the tissue. Examples of movements that may cause the nervous tissue to move with respect to the implanted electrode are breathing movements, pulsations in blood pressure or body movements such as rotation of the head. To reduce the severity of the tissue reactions, the electrode needs to be as mechanically compliant with the target soft tissue as possible to be able to move with pulsative and other movements in the tissue thereby reducing the tearing forces between implanted electrode and tissue. For this reason, highly flexible microelectrodes (here defined as electrodes with a diameter less than about 100 µm) have been developed. To precisely implant highly flexible microelectrodes without substantial deviation from intended track lines, structural support during insertion is necessary. Several methods providing structural support during insertion to deep structures are known by the art such as transiently attaching the microelectrodes to a rigid support rod with a dissolvable glue or by embedding the microelectrodes in a hard but dissolvable material.

WO 2017/095288 discloses a proto microelectrode comprising individual microelectrodes and a biocompatible solid support material. The solid support material sufficiently stabilizes the proto microelectrode to allow implantation into soft tissue. According to an embodiment the microelectrodes can be disposed to fan out in distal direction. The individual microelectrodes are firmly embedded into the solid support material. WO 2017/095288 fails to disclose a solid support material providing a continuous lumen trough the solid support material. The microelectrodes are further firmly embedded into the support material which prohibits the microelectrodes to move with respect to the solid support.

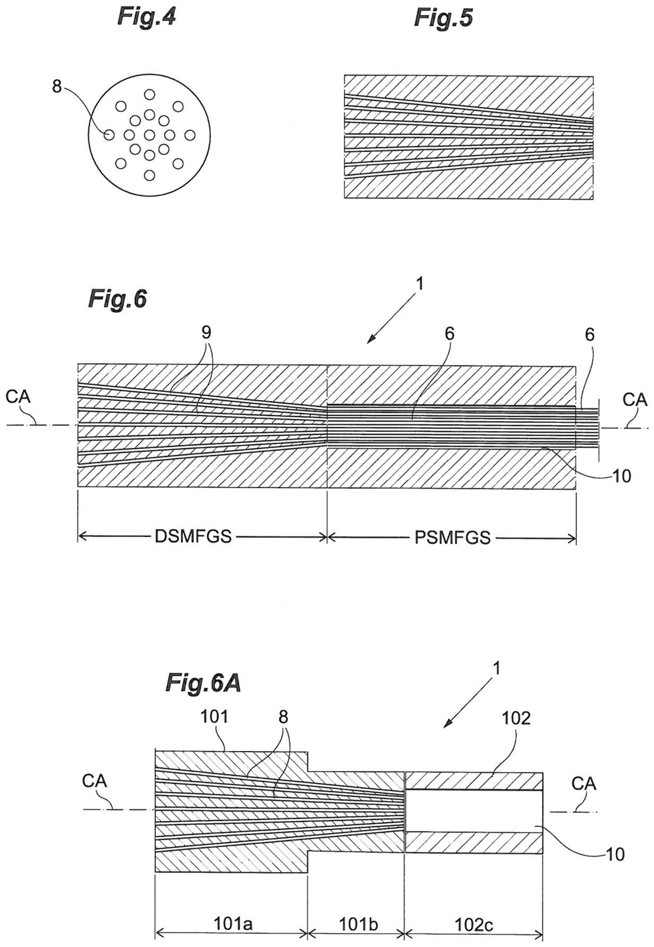

US 2019/0298993 relates to a neural probe comprising an electrode assembly with simulation and recording electrodes positioned on a very specific carrier similar to a carpenter's tape spring. The tape spring-type carrier provides the electrode assembly with stiffness along a line of trajectory once deployed into body tissue, but with a degree of flexibility that allows the electrode assembly to move with the tissue. Each thin-film neural probe electrode assembly comprises multiple metal traces and sites. These electrode assemblies cannot be precisely introduced into tissue without a carrier. As further elaborated, the probe is not rigid but has a degree of stiffness provided by the tape spring-type carrier (inside the probe) that maintains a desired trajectory into the body but will subsequently allow the probe to flex and move unison with the movement of the body tissue. Further, the probe contains a guide tube which allows for the deployment of the carrier in a three-dimensional arrangement. As disclosed the guide tube is made of a rigid material that can be inserted into tissue without buckling and can maintain a generally straight trajectory through the tissue. The material of the guide tube is plastic, such as medical grade plastic, or metal, e.g. titanium. The carrier has the form of a tape spring-type carrier in its unstressed condition, fig 4B. The carrier is forcibly bent by the configuration of the channels of the guide tube. At the bend the carrier under stress is locally transformed into a linear cross-section, fig. 4A. As the carrier has passed through a bend of a channel the stressed condition of the carrier is transformed back to its unstressed concave cross-section, fig. 4B. Bending and keeping a tape spring-type carrier with a concave cross-section area bent necessitates a rigid guide tube. Without a rigid guide tube the tape spring-type carrier will have an intrinsic desire to gradually return to its unbent, unstressed linear configuration. Thus, without the presence of a stable guiding tube the carriers would exhibit a constant pressure on the adjacent tissue, that risk triggering inflammation and eventually potential encapsulation of the carriers and electrode assemblies significantly impairing the functionality of the electrodes over time. To replace the non-transient material of the guide tube with a material which is dissolvable and/or degradable would not only risk compromise the bending of the unstressed carrier during insertion through the channels of the guide tube but would also fail to keep the bent stressed section of a carrier in its proper configuration after dissolution of the guide tube. With the dissolution of the guide tube the carriers would exert a force on the adjacent tissue which over time would impair the functionality of the neural probe. Moreover, it is described that the guide tube includes bent channels and given that these are part of the guide tube it can be inferred that they are of same materials. The described channels are bent relatively rigid tubules anchored to the guide tube which, in turn, is intended to be anchored to the skull. The guide tube cannot be explanted without also explanting the electrodes, since the holes in the guide tube will simultaneously pull out the electrodes. So, this construction will stay in tissue and there is nothing in the description that suggest otherwise. Such a structure if resident in tissue would likely cause substantial tissue reactions that may affect the tissue at a distance.

The combination of a tape spring-type carrier and the disclosed guide tube would lead to significant rupture of the carriers with respect to the surrounded soft tissue should the guiding tube be contemplated to be removed from the soft tissue while leaving the carriers behind in the soft tissue.

Specific spatial regions of the brain are usually associated with distinct functions and the functions may shift abruptly over very short distances such as over a distance of 100 µm. Due to inter-individual innate anatomical differences, use dependent changes, age dependent changes or changes due to degenerative disease, the precise location of a certain function may differ between individuals. It is therefore usually not possible to predict in advance the exact coordinates in the brain for optimal placement of electrodes. For microelectrodes, this is problematic since the range from which neuronal activity can be recorded with sufficient signal to noise ratio is very limited (typically within less than 70 µm radius from electrode contact). Microelectrodes also have a relatively limited capacity to eject current within prevailing safety limits which means they need to be placed as close to the target neurons as possible.

By providing a surplus of highly flexible, and therefore biocompatible, microelectrodes in the target region, evaluating which of them provide therapeutic effects and which produce un-wanted side effects on stimulation, then selecting a subgroup of microelectrodes within the implanted cluster that together provide effective symptom relief during stimulation without significant side-effects, an increased precision of brain stimulation with less side effects can be accomplished (Forni et al, 3D Microelectrode Cluster and Stimulation Paradigm Yield Powerful Analgesia without Noticeable Adverse Effects, Science Advances 7 (41), 2021). However, finding subgroups of appropriate microelectrodes within a cluster of microelectrodes requires iterative testing which can be very time consuming. To be able to systematically search for the location of the best group of implanted microelectrodes and, to provide maps in 3D of therapeutically effective sites and sites provoking adverse effects related to the brain anatomy and corresponding atlases that can guide the search in subsequent subjects/patients and also to locate the source of neuronal signals using multiple cooperating microelectrodes, there is a need for insertion methods that improve the regularity of the spacing of flexible microelectrodes in the target.

While single or a few highly flexible microelectrodes can be inserted deeply into sodt tissue if e.g. transiently attached to the circumference of a rigid support structure, the injury caused by the insertion is increased substantially by the support structure. On implanting many microelectrodes the time consumption for the surgical procedure will increase with number of microelectrodes. It is therefore advantageous if the individual microelectrodes can be inserted substantially simultaneously through the same guiding system. If guided by a syringe, precise placement of each of a plurality of microelectrodes in the tissue after being expelled from the opening of the syringe is problematic since highly flexible electrodes may for example adhere to each other in the syringe and to the inner wall of the syringe while being advanced out from the syringe, and may therefore spread more or less randomly. This makes it very difficult and time consuming to determine the brain coordinates for respective microelectrode using e.g. imaging. This impedes calculation of sources of brain signals when used for recordings and can also result in increased time consumption to find optimal microelectrodes for stimulation in therapeutic or mapping purposes.

To at least partially overcome the problem of how to precisely implant ultra-flexible microelectrodes in relation to the functions to be studied or the functions to be affected by stimulation, methods have been developed in which bundles of microelectrodes are initially separated and stabilized by hard but dissolvable materials during the insertion and, as the dissolvable material is swelling/dissolving the bundles are further advanced into the target area and spread as a cluster of microelectrodes (WO 2007/040442, WO 2017/095288). However, the track lines and separation of the individual microelectrodes in the bundle during insertion depend on a number of parameters such as swelling and dissolution time course for the dissolvable materials that can be different for inner and outer parts of the dissolvable material. This may cause the outer parts to swell/dissolve earlier than the inner parts. Irregularities in the dissolvable material may also contribute to non-homogenous swelling. Moreover, the swelling and dissolution time course often depend on for example brain temperature and accessibility of body fluids around the probe that can show regional differences in the brain and also differences between individuals.

A related problem is how to regularly and effectively implant flexible microtubes to administrate drugs or living cells into defined regions in the soft tissue over longer time periods such as days, weeks or months. For example, stem cells intended for therapeutic uses are usually injected slowly from a single syringe into target tissue as the syringe is slowly retracted. To get a more evenly distributed delivery, several tracks are made one after the other. This is very time consuming and may also result in loss of stem cells as well as increase the damage of the tissue. A similar problem is to provide for an even distribution of a drug in soft tissue for example when addressing a tumor.

While known channel guiding systems attachable on the skull are known for insertion of electrodes, they need to be withdrawn after surgery, which risk dislocating the implanted electrodes and also increase the risk for additional injury.

Generically, the invention inter alia relates to a proto microfilament assembly comprising a microfilament guiding structure, an elongated hollow guiding member, a pin and microfilaments and a cover structure

### OBJECTS OF THE INVENTION

A purpose with the present invention is the provision of a guiding system for insertion of flexible microfilaments such as flexible microelectrodes or flexible catheters.

A further purpose with the present invention is the provision of the simultaneous precise placement of a cluster of flexible microfilaments.

A further purpose with the present invention is the provision of the simultaneous precise placement of a cluster/array of flexible microfilaments without the need for attaching the flexible microfilaments to a support structure along their distal length introduced into soft tissue (such as a carrier).

A further purpose is the provision of improving the spatial precision of the simultaneous placement of a cluster of flexible microfilaments in soft tissue while reducing tissue injury caused under insertion

A further objective is the provision of precise placement of a cluster/array of flexible microfilaments and the subsequent removal and dissolution and/or disintegration of as many parts of the proto microfilament assembly as possible.

A further objective is the reduction or even substantial reduction of long-term tissue reactions in soft tissue caused by the placement and presence of a cluster of flexible microfilaments in soft tissue which can impair the functionality of the cluster/array of flexible microfilaments.

Another objective is the provision of an electrode assembly improving the spatial resolution of recordings and stimulations in soft tissue.

A still further objective is the provision of a highly ordered cluster/array of microfilaments.

A still further objective is to enable verification of the intended microfilament cluster/array before implantation.

Further objectives are evident or may be extracted from the description and claims.

### INTRODUCTION OF THE INVENTION AND EXPLANATION OF CONCEPTS AND TERMS

The present invention discloses new guiding tools, such as a microfilament guiding structure for simultaneous insertion of a plurality of flexible microfilaments, into soft tissue that at least partly overcome some of the problems of precisely implanting them in soft tissue.

The invention relates to a proto filament assembly comprising at least the following constructional features: a microfilament guiding structure, microfilaments, an elongated hollow guiding member and a pin.

The proto filament assembly may also comprise a hollow supporting guide (embodiment Δ).

The invention further relates to a microfilament guiding structure.

Furthermore, the invention also relates to methods specifically for the manufacture of the microfilament guiding structure.

Additionally, the invention also encompasses a method for the placement of a cluster/array of microfilaments.

A microfilament can be a microelectrode, microtubule, optical microfibers or any other elongated microfiber. Two types of microfilaments are specifically contemplated: hollow microfilaments and microelectrodes comprising electrically conductive filaments, herein referred to as electrically conductive microelectrodes, or simply microelectrodes. Preferably, the material (or materials) of the microfilament has a level of internal tension(s) that prevents the microfilament to substantially deviate from a linear trajectory in soft tissue on exiting the channels of the filament guiding structure while being propelled forward into the soft tissue. The microfilaments are preferably characterized as having a straight conformation, i.e. they do not exhibit bends that would tend to provide a curved path in soft tissue on propelling the provided microchannels out from the guiding system and into the tissue. The rational is to enable a precise placement in the tissue. Furthermore, the microfilament has preferably a flexibility sufficient for accommodating movement of soft tissue, such as neural tissue, without significantly affecting the soft tissue. Preferably, the microfilament, or at least the part (most distal section) of the microfilament or at least part of the microfilament adjacent and in direct contact with soft tissue, possess a level of internal tensions that prevents the microfilament to substantially deviate from the trajectory provided by the channels of the filament guiding structure during insertion into soft tissue, and having a flexibility sufficient for accommodating movement of soft tissue, such as neural tissue, without significantly affecting the soft tissue.

The term 'transient' characterizes structures and members (entities) which gradually dissolves and/or disintegrates, degrades in body (tissue) fluid. The terms 'distal' and 'proximal' are used to denote sections, regions, parts (or any further equivalent term) of the proto filament assembly specifically the microfilament guiding structure, the elongated hollow guiding member and the optional pin guiding structure. Distal denote a section, region, etc. which is located deeper inside soft tissue than a proximal section, region. The terms distal and proximal are not absolute in position but are relative.

The term "flexible microfilaments" is defined as microfilaments that do not themselves possess sufficient rigidity to be implanted all the way to a target tissue without bending away from the intended track line and that, consequently, need structural support in case a precise insertion is intended.

The term 'microfilament guiding structure' relates to a structure comprising channels (lumens) for each microfilament. Also, at least in the distal section of the microfilament guiding structure the radial distances between at least some the channels gradually increases in distal direction. The microfilament guiding structure is typically positioned at the distal end of the elongated hollow guiding member. According to an embodiment Δ, the microfilament guiding structure is positioned with respect to the elongated hollow guiding member such that a distal part of the microfilament guiding structure is disposed distally to the elongated hollow guiding member (outside to the hollow guiding member) while a proximal part of the microfilament guiding structure is disposed inside (within) the elongated hollow guiding member. In the context of the embodiment Δ, the term 'part' is used instead of 'region' with respect to the microfilament guiding structure.

The term 'pin' denotes a structural member to which the microfilaments are removably secured. The pin may be configured such that there is a gap between the pin and the elongated hollow guiding member enabling sliding while minimizing lateral movement of the pin. The pin can be made of a transient material dissolvable in body fluids. Should the pin be made of transient materials the dissolution profile must be substantially slower that the dissolution of the microfilament guiding structure and optionally the hollow supporting guide or optional pin guiding structure. Preferably, the pin is made of a material which maintains its structural rigidity over time. Suitable pin materials can be selected from metals, metal alloys, polymers and fiber reinforced polymers (carbon fiber). According to an embodiment, the pin may be removably attached to a pin guiding structure.

The term 'pin guiding structure' is a structure which secures the pin to an essentially central position (coinciding with the central axis of the elongated hollow guiding member) with respect to the elongated hollow guiding member as long as the pin is attached to the pin guiding structure specifically when the pin and pin guiding structure' is moved in distal direction. The pin guiding structure is also configured to be able to slide inside the elongated hollow guiding member in distal (axial) direction. The pin guiding structure preferably dissolves and/or disintegrates in body fluids.

The term 'hollow supporting guide' denotes a transient guide of the embodiment Δ situated between the microfilament guiding structure and the pin. The hollow supporting guide preferably dissolves and/or disintegrates in body fluids.

The present invention is also based on the insight that also flexible microfilaments can be propelled (inserted) for a short distance (the distance depend on insertion speed, flexibility and degree of straightness of the microfilament) into soft tissue without substantial deviation from intended track provided that they do not have to traverse mechanically tougher tissues such as meningues such as pia mater and arachnoidea or other tissues providing structural support for soft tissue.

The present invention is further based on the insight that a microfilament guiding structure (used in conjunction with an elongated hollow guiding member) for orderly insertion of highly flexible microfilaments should be implanted relatively close to the target tissue, such as between 1 to 20 mm, or preferably 5 to 12 mm from the targeted tissue, to provide a precise implantation. To reduce tissue injuries the guiding structure might be implanted through a narrow track comprising said gel track provided by the technology presented in WO 2016/032384 A1.

The present invention is also based on the insight that such an implantable microfilament guiding structure should disintegrate once the microelectrodes have been propelled out into the target tissue, thereby eliminating the need for explanting the filament guiding structure from the tissue.

The microfilament guiding structure denotes a structure found in the distal part of the proto filament array. The function of the microfilament guiding structure is to provide a predetermined spatial arrangement of microfilaments localized within channels (which may also be denoted as filament guiding channels or guiding channels) of the microfilament guiding structure. It is also important that the microfilaments accommodated by the channels can be moved essentially independent of the microfilament guiding structure. A further feature of the disclosed microfilament guiding structure is that the channels of the microfilament guiding structure exhibit a spatial configuration of at least some of the channels such that the distance between the distal exit of a channel and the center axis of the filament guiding structure is longer than the distance between the proximal exit of said channel and the center axis of the filament guiding structure.

The microfilament guiding structure is preferably of a material or materials which are transient and, hence, a material gradually disintegrating and absorbed by soft tissue fluids. The microfilament guiding structure may comprise materials which essentially preserve the configuration over time. Such materials should preferably be flexible (e.g. silicone). To use transient material for the microfilament guiding structure is more preferred than using non-transient materials. This spatial configuration of the channels provides that the separation of individual microfilaments increases as the microfilaments are moved/propelled with respect to the microfilament guiding structure in distal direction. The independent movement of the individual microfilaments with respect to the microfilament guiding structure and out of the filament guiding structure in distal direction provides for the formation of a cluster/array of microfilaments in targeted tissue with a wider spatial distribution than the spatial distribution of the filament guiding structure. The propelling of the microfilaments from the filament guiding structure into targeted tissue also ensures that each filament is surrounded by tissue at its distal part. The microfilament guiding structure is also a prerequisite for the placement of a cluster of microfilaments into targeted soft tissue without the need of additional structural support from stiff extensions (carriers supporting microfilaments adjacent to soft tissue) substantially increasing the probability of tissue damage. By using a proto microfilament assembly comprising the microfilament guiding structure at its distal end, the microfilament guiding structure can be very precisely positioned to the targeted area of the soft tissue prior to the propelling of the microfilaments in distal direction thereby providing an array of microfilaments in the targeted area. The microfilament guiding structure also ensures that each microfilament will be surrounded by tissue in its distal part directly after insertion.

The microfilament guiding structure may comprise a further structural feature in the proximal section. A microfilament guiding structure comprising a further proximal structural feature may be characterized as comprising a distal and proximal section. The distal section or part of the microfilament guiding structure is the section or part characterized by a) a number of channels equal to or higher than the number of microfilaments, i.e. each microfilament is accommodated by a separate guiding channel, and/or b) that the distance between at least some of the individual channels continuously increase in distal direction. In the proximal section of the microfilament guiding structure the distances between individual channels accommodating the microfilaments remain essentially similar in axial direction. The proximal section of the microfilament guiding structure may also be characterized by comprising a fewer number of microfilament channels than present in the distal section. According to an embodiment, the proximal section of the microfilament guiding structure comprise one channel for the accommodation of all microfilaments, this channel suitably positioned centrally within the proximal section of the microfilament guiding structure.

According to an embodiment, at least one channel of the microfilament guiding structure may comprise more than one microfilament, especially if such microfilaments are microelectrodes.

According to an embodiment, the distal section of the microfilament guiding structure does not comprise multiple channels, but one channel accommodating all microfilaments. If the microfilament guiding structure is set together of two discrete structures, a distal and a proximal, in some embodiments also referred to as distal microfilament guiding sub-structure and proximal microfilament guiding sub-structure, the proximal microfilament guiding sub-structure preferably comprises one central conduit with a size accommodating all microfilaments. The proximal microfilament guiding sub-structure comprising one central conduit is in some embodiments referred to as hollow supporting guide.

If the microfilaments in the gap between the microfilament guiding structure and the pin be bundled together with a transient rigidity providing material, e.g. glue and/or any of the dissolvable or degradable materials disclosed herein, then either the proximal section of the microfilament guiding structure should comprise a central conduit/channel with a dimension capable of accommodating the bundle of microfilaments, or, a hollow supporting guide as disclosed herein should be interposed between the microfilament guiding structure and the pin.

According to an embodiment of the proto microfilament assembly, at least one channel of the microfilament guiding structure comprised in the assembly may comprise more than one microfilament, especially if such microfilaments are microelectrodes.

The term 'region' and 'part' is used in conjunction with the microfilament guiding structure. The term 'part' is only used in conjunction with the microfilament guiding structure related to the embodiment Δ where the microfilament guiding structure is partly disposed outside (distally) of the elongated hollow guiding member. According to an embodiment, the microfilament guiding structure may have at least two regions, a distal region and proximal region, differing in respect of radial extension. According to an embodiment, the term section and region may be equivalent. For this embodiment of the microfilament guiding structure the channel feature and the radial extension feature coincide. Thus, the distal section is equivalent to the distal region, the proximal section is equivalent to the proximal region. According to an embodiment of the microfilament guiding structure the section and region are overlapping. For example, the distal section may comprise a distal region with one radial extension and a proximal region with a different radial extension.

The proto filament assembly comprises a pin guiding structure, alternatively, a pin positioned proximally to the microfilament guiding structure. The pin guiding structure is made of any transient material or materials which are suited for the microfilament guiding structure. According to an embodiment, the transient materials of the microfilament guiding structure and the pin guiding structure may be same, but may also be different. The pin guiding structure may comprise a rigid pin suitably centrally located in the pin guiding structure and extending from the pin guiding structure in proximal direction. Further, the microfilaments are typically accommodated centrally in the pin guiding structure, suitably around the rigid pin. The microfilaments extend from the pin guiding structure in proximal direction. According to an embodiment of the proto filament assembly, embodiment Δ, the pin guiding structure is omitted. Instead of having the pin attached to the pin guiding structure the pin is configured to slide inside the elongated hollow guiding member. Preferably, there is a gap between the pin and the elongated hollow guiding member enabling sliding and minimizing lateral movement of the pin. This Δ embodiment also comprises a hollow supporting guide disposed between the microfilament guiding structure and the pin.

An embodiment relates to a proto microfilament assembly having a distal region and a proximal region comprising the microfilament guiding structure as defined herein, further comprising microfilaments disposed in the channels of the microfilament guiding structure, an elongated hollow guiding member, and a pin, where the microfilaments from the microfilament guiding structure extend in proximal direction, the microfilaments being removably (transiently) attached to the pin, the pin being located proximally to the microfilament guiding structure and being configured to be slidably disposed inside the elongated hollow guiding member, the proto filament assembly being sufficiently stiff to be inserted into a targeted area of soft tissue, wherein the pin is disposed inside the elongated hollow guiding member with respect to the microfilament guiding structure to provide a gap between the microfilament guiding structure and the pin, the microfilament guiding structure being transiently attached to the elongated hollow guiding member, wherein the microfilaments can be propelled outside of the channels in distal direction by moving the pin in distal direction without essentially moving the elongated hollow guiding member and the microfilament guiding structure.

A further embodiment of the proto microfilament assembly relates to a microfilament guiding structure comprising a distal part and a proximal part, the distal part of the microfilament guiding structure disposed distally to the elongated hollow guiding member and the proximal part being disposed inside the elongated hollow guiding member.

Yet a further embodiment of the proto microfilament assembly relates to a hollow supporting guide between the microfilament guiding structure and the pin and disposed within the elongated hollow guiding member, the hollow supporting guide comprising a central conduit accommodating the microfilaments, the pin being disposed inside the elongated hollow guiding member with respect to the hollow supporting guide to provide a gap between the hollow supporting guide and the pin.

Yet a further embodiment of the proto microfilament assembly provides for that the structural rigidity of the microfilaments is enhanced along a section between the hollow supporting guide and the distal end of the pin prohibiting or essentially prohibiting individual microfilaments to bend or buckle when the pin is moved in distal direction.

An embodiment of the proto microfilament assembly provides for that the microfilaments are fully disposed inside the channels of the microfilament guiding structure before the engagement of the pin in distal direction.

The Δ embodiment relates to a proto assembly for the pre-positioning of a microfilament guiding structure in the vicinity of a targeted spatial location within soft tissue, e.g. neural tissue, the proto assembly comprising a microfilament guiding structure, an elongated hollow guiding member, a hollow supporting guide, a pin, and at least two microfilaments. The elongated hollow guiding member is preferably circular. As elaborated, a distal part of the microfilament guiding structure is disposed distally to the elongated hollow guiding member (outside to the elongated hollow guiding member) while a proximal part of the microfilament guiding structure is disposed inside (within) the elongated hollow guiding member. Furthermore, the microfilament guiding structure is secured to the elongated hollow guiding member which enables the microfilaments disposed inside the channels of the microfilament guiding structure to be propelled into the soft tissue in distal direction without significant axial movement of the microfilament guiding structure. The microfilament guiding structure may be attached to the elongated hollow guiding member by a suitable biocompatible glue degradable or dissolvable in body fluid such as any one of the glues disclosed herein. An alternative attachment of the microfilament guiding structure to the hollow guiding member can be by threads comprised in the microfilament guiding structure and attached or anchored by suitable means at proximal region of the elongated hollow guiding member. The attachment may be made physically or by means of a glue which may be degradable. The microfilament guiding structure may comprise a plurality of threads, such as at least 2, 3, 4, 5, 6, or more. The threads may be integrated within a substantial axial length of the microfilament guiding structure. The threads suitably exit the microfilament guiding structure laterally before the proximal end of the microfilament guiding structure. This embodiment Δ also comprises a hollow supporting guide disposed between the microfilament guiding structure and the pin. The hollow supporting guide is preferably positioned in close vicinity to the microfilament guiding structure, preferably the hollow supporting guide is disposed such that the hollow supporting guide touches the microfilament guiding structure. The pin is positioned with respect to the elongated hollow guiding member such that it can slide while the lateral (radial) disposition with respect to the central axis (of the elongated hollow guiding member) remains essentially same. The pin may be disposed with a small gap between the pin and the elongated hollow guiding member enabling sliding and minimizing lateral movement of the pin. Between the distal end of the pin and the proximal end of the hollow supporting guide there is a gap. In order for the microfilaments to be propelled from a position inside the channels of the microfilament guiding structure into the soft tissue the pin must have the ability to be moved axially in a distal direction. It is preferred that in the proximal part of the microfilament guiding structure the microfilaments are disposed along the central axis, suitably such that the radial distances of the individual microfilaments remain similar. Preferably, the microfilaments from the proximal exit of the microfilament guiding structure to the distal end of the pin are axially aligned, suitably along the central axis of the elongated hollow guiding member. The hollow supporting guide is preferably circular with an outer diameter enabling the disposition in the elongated hollow guiding member. The diameter of the central channel in the hollow supporting guide has suitably a dimension capable of accommodating all microfilaments and safeguarding that the individual microfilaments have sufficient lateral support not to significantly buckle when moved in distal direction. At least along a distal portion of the hollow supporting guide all microfilaments are not attached to one another as each microfilament have been allocated a unique channel inside the microfilament guiding structure. Should the microfilaments be attached to one another in the proximity of where individual microfilaments are disposed within respect to their unique channel the movement of individual microfilaments in the channels in the distal direction would be hindered. It is further preferred that the bundle of microfilaments, at least between the proximal end of the hollow supporting guide and distal end of the pin comprises at least an additional component providing rigidity preferably in axial direction. The rigidity providing component may be any of the dissolving and/or disintegrating materials disclosed herein. In this embodiment Δ the pin has preferably a recess where the microfilaments are disposed. This recess may extend over a part of the pin and if so the recess preferably extends over the distal part of the pin from the distal end up to a location where the microfilaments deviate in radial (lateral) direction away from the pin and also optionally away from the elongated hollow guiding member. The recess may also extend over the entire length of the pin. The rigid pin may have an annular cross-section with a lateral partition, gap, preferably having a radial extension of from 5° to 180°, preferably from 10° to 170, preferably from 15° to 170°. In the pin recess or gap the filaments are preferably disposed. The filaments in the recess may be bundled by any suitable measure, e.g. with any of the glues disclosed herein. Preferably, microfilaments are securably attached to the distal end of the pin with any of the glues disclosed herein. This embodiment Δ is preferably combined with the embodiment of the proto assembly comprising the deviation of the filaments in lateral direction. If microfilaments are essentially deviated in lateral direction with respect to the central axis of the elongated hollow guiding member a pin with a recess is particularly preferred.

According to an embodiment, the proto filament assembly may comprise an outer cover or cover structure serving the purpose of delaying the entry of body fluids specifically into the channels of the microfilament guiding structure and, hence, delaying the dissolution and/or disintegration of any structure or material of the proto filament assembly comprising a substance dissolving and/or disintegrating when in contact with body fluids. The cover may comprise three layers, where the middle layer acts as a liquid-resistive barrier. The layer facing (or closest to) the elongated hollow guiding member and the layer adjacent to the surrounding soft tissue (when inserted into siot tissue) comprises any of the herein disclosed material which dissolves and/or disintegrates, and gel-forming materials. It is important that the distal part of the cover does not comprise a liquid-resistive barrier. Preferably, the liquid-resistive barrier does not extend in distal direction from the distal end of the microfilament guiding structure to not hinder the movement of microfilament out from the microfilament guiding structure. The cover may comprise an elongated member such as a wire, strand, thread suitably integrated into liquid-resistive barrier disposed in axial direction. This elongated member of the cover can be used for ripping the cover apart when the cover is removed from the soft tissue.

According to another embodiment the proto microfilament assembly may be covered by a water-resistant layer. Such a layer may comprise any one of the dissolvable are degradable materials disclosed herein.

The proto filament assembly is preferably inserted into soft tissue in conjunction with a stereotaxic instrument. More specifically, the proximal part of the proto filament assembly may be positioned inside a stereotaxic guide of a stereotaxic instrument.

According to an embodiment, the proto filament assembly may be configured such that the microfilaments proximally to the pin guiding structure are not disposed essentially around the central axis inside of the proto filament assembly but are, from a location proximally to the pin guiding structure and in proximal direction, disposed outside the proto filament assembly and where applicable also outside of the stereotaxic guide, suitably essentially parallel to the central axis of the proto filament assembly or stereotaxic guide. The microfilaments are preferably secured to a member which may be secured to a tissue, such as bone tissue, in relation to which tissue the soft tissue is able to spatially move. To have the microfilaments disposed outside the proto filament assembly in the proximal part provide for a greater spatial freedom of configuring the microfilaments. The microfilaments outside the proto filament assembly may along a section be configured to improve the intrinsic ability of the microfilaments to accommodate for spatial movement of the soft tissue, in particular soft tissue movement essentially parallel to the central axis of the filament assembly (i.e. filament assembly after dissolution of e.g. filament guiding structure, pin guiding structure and optional removal of elongated hollow guiding member should the hollow guiding member be resistant to dissolution in body fluids).

The proto filament assembly comprises an elongated hollow guiding member. The microfilament guiding structure and the pin guiding structure are accommodated by the elongated hollow guiding member. The elongated hollow guiding member provides several functions. One function is to position the microfilament guiding structure in soft tissue prior to the insertion of the microfilaments into the targeted tissue. A further function is to sufficiently anchor or enable anchoring of the microfilament guiding structure to the elongated hollow guiding member. The anchoring of the microfilament guiding structure to the elongated hollow guiding member is important because the axial movement of the individual microfilaments inside the channels in distal direction should be accomplished without significant movement of the microfilament guiding structure. A further function of the elongated hollow guiding member is to provide sufficient structural rigidity to the proto filament assembly to allow a precise axial movement during the insertion of the proto filament assembly into soft tissue. A further function of the elongated hollow guiding member is the provision of an axial movement of the pin guiding structure in distal direction. The enablement of the axial movement of the pin guiding structure while the microfilament guiding structure is essentially anchored to the elongated hollow guiding member necessitates the presence of a gap between the microfilament guiding structure and the pin guiding structure.

The elongated hollow guiding member can have any configuration as long as the following criteria are meet: proper axial alignment of the microfilament guiding structure and the pin guiding structure; anchoring of the microfilament guiding structure (or at least the distal microfilament guiding sub-structure); enablement of axial movement of pin guiding structure or the pin; sufficiently rigid to allow precise positioning of the proto assembly and specifically the microfilament guiding structure and as a consequence the array/cluster of microfilaments at the targeted tissue;

The elongated hollow guiding member is typically rotationally symmetric. Any rotational symmetric form is plausible. Various rotationally symmetric forms will provide different surface areas. It is preferred to choose rotational symmetric forms providing smaller surface area. Therefore, elliptical and cylindrical forms are preferred.

According to an embodiment, the elongated hollow guiding member has an annular cross-section comprising a lateral partition, i.e. gap or opening, along the entire axial length of the elongated hollow guiding member. Preferably, the lateral partition can have a radial extension from about 5° to about 180°, from about 5° to about 170°, from about 5° to about 160°, from about 5° to about 90°. Preferably, the lateral partition can have a radial extension from about 5°, 10°, 15°, 20°, 25°, 30°, 35°, 40°, 45°, 50°. Preferably, the lateral partition can have a radial extension up to about 60°, 65°, 70°, 75°, 80°, 85°, 90°, 95°, 100°, 105°, 110°, 120°, 130°,140°, 150°, 160°, 170°, 180°. Any one of the low ranges may be combined with any one of the high ranges. The radial extension of the gap can vary along the length of the elongated hollow structure. An example is that the extension of the gap is smaller distally and proximally that at an intermediate region of the elongated hollow structure. According to an embodiment, the elongated hollow guiding member can comprise a continuous gap from the most distal location to a location where the microfilaments deviate from the assembly in radial direction. Furthermore, the radial extension of the lateral partition of the distal section of the elongated hollow guiding member may be smaller than the radial extension of the lateral partition of a section proximally to the distal section of the elongated hollow guiding member.

According to a further embodiment, the elongated hollow guiding member has an annular cross-section comprising a lateral partition in axial direction along a portion of the axial length of the elongated hollow guiding member preferably a continuous gap from the distal end to the location where the microfilaments are deviated laterally if applicable. The lateral partition in axial direction may be positioned along the distal section of the elongated hollow guiding member.

The elongated hollow guiding member is made of a material which either is of a non-transient material for which the structural properties are not structurally affected by soft tissue (or body liquids), or a material which is transient but disintegrates substantially slower than the materials of the microfilament guiding structure of the hollow support guide and of the pin guiding structure. The elongated hollow guiding member may also be characterized as having a distal and proximal region. The division of the elongated hollow guiding member in the distal and proximal region may be governed by the configuration of the annular cross-section of the elongated hollow guiding member and more specifically the width (angular width) of the lateral partition. According to an embodiment, the distal region of the elongated hollow guiding member has a width of the partition which is smaller than the width of the lateral partition of the proximal region of the elongated hollow guiding member. According to an embodiment, the proximal region of the elongated hollow guiding member has a lateral partition with a width corresponding to an angular extension of about a semi-circle (about 180 deg). The distal lateral partition may have a width corresponding to an angular extension of between about 5 to about 30 deg. According to an embodiment the axial length of the distal region of the microfilament guiding structure is essentially similar to the axial length of the distal region of the elongated hollow guiding member.

The term 'proto microfilament assembly' denotes a proto microfilament assembly which has not yet been inserted into soft tissue or which has not yet assumed the configuration of a filament array in the soft tissue. Thus, once the proto filament assembly is correctly positioned within soft tissue and the microfilaments have been propelled into the targeted tissue, the proto filament assembly gradually evolves into a filament array comprising a bundle of microelectrodes fanning out within the soft tissue as a cluster/array of microfilaments once any non-transient part has been removed, typically elongated hollow guiding member and pin.

A further feature of the proto microfilament assembly is that the microfilament needs to have a conformation which is capable of transferring axial forces exerted by the pin to which the microfilaments are attached when moved distally and thereby moving the individual microfilaments accommodated within the channels of the microfilament guiding structure in a distal direction. The microfilaments are preferably bundled together in the section defined by the gap between the microfilament guiding structure and the pin or in some embodiments the pin guiding structure. The bundling of the microfilaments per se increases general rigidity but can be further improved by the application of a material further improving the rigidity. The term rigidity implies the capability of translating axial forces without significant bending.

A rigid pin is optionally secured to a pin guiding structure. The pin is suitably disposed inside the elongated hollow guiding member and should extend out of the elongated hollow member proximally. The pin may be constituted of non-transient materials including metals (e.g. metal alloys) and reinforced curable resins (e.g. carbon fiber) and transient materials albeit more resistant to degradation in body fluids than the pin guiding structure and the microfilament guiding structure. The rigid pin may be configured to have an annular cross-section and comprising a lateral partition of the annular cross-section. The lateral partition may extend axially along a specific length of the rigid pin or along the entire length of the rigid pin. This partition is preferably configured such that it can harbor the microfilaments. The partition also allows for efficient attachment of the microfilaments (microfilament bundle).

The term 'soft tissue' includes nervous tissues found in the brain, spinal cord, endocrine organs, and tissue of muscles and connective tissue. The invention may be applicable to nervous tissue, specifically tissue of the nervous system including the brain, spinal cord and the central nervous system.

### SHORT DESCRIPTION OF THE FIGURES

In some of the figures letter D and P are indicated in the vicinity of arrows. D and P stands for Distal and Proximal, respectively. An arrow in the vicinity of D illustrates the distal direction, an arrow in the vicinity of P illustrates the proximal direction.
Fig. 1 Illustrates a schematic figure of a proto microfilament assembly. For clarity the microfilament are not shown.
Fig. 2 illustrates a schematic view of a microfilament guiding structure.
Fig. 2a illustrates a cross-section at A-A of the distal region of a microfilament guiding structure.
Fig. 2b illustrates a cross-section at B-B of the proximal region of a microfilament guiding structure.
Fig. 3 illustrates a schematic view of an elongated hollow guiding member.
Fig. 3a illustrates a cross-section at C-C of the distal region of an elongated hollow guiding member.
Fig 3b illustrates a cross-section at D-D of the proximal region of an elongated hollow guiding member.
Fig. 4 illustrates an axial view of the distal side of a microfilament guiding structure further depicting the spatial arrangement of some filament guiding channels.
Fig. 5 illustrates a lateral cross-section of a microfilament guiding structure depicting individual guiding channels.
Fig. 6 illustrates a lateral cross-section of a microfilament guiding structure having distal section and a proximal section. The proximal section comprises one central channel for the accommodation of the individual microfilaments.
Fig. 6A illustrates a lateral cross-section of a microfilament guiding structure and a hollow supporting guide as implemented in e.g. the proto microfilament assembly as disclosed by embodiment Δ.
Fig. 6B illustrates a lateral cross-section of a microfilament guiding structure and a hollow supporting guide as implemented in e.g. the proto microfilament assembly as disclosed by embodiment Δ but with a different configuration of the channels of the microfilament guiding structure.
Fig 7 illustrates a lateral cross-section of a microfilament guiding structure having distal section and a proximal section. The proximal section comprises one central channel for the accommodation of the individual microfilaments. The spatial arrangement of the channels is different than the spatial arrangement illustrated by fig. 6.
Fig. 8 illustrates the definition of angle α used for characterizing the spatial orientation of guiding channels within the microfilament guiding structure (the distal section of the microfilament guiding structure).
Fig. 9 illustrates a schematic presentation of the pin guiding structure including the rigid pin and
Fig.10 and fig 11 illustrate cross-sectional views of the proto microfilament assembly of fig. 1 at positions E-E and F-F.
Fig. 12 illustrates a channel positioning member used in a microfilament guiding structure manufacturing process.
Fig, 13. Illustrates the placement of channel-forming rods/wires between two channel positioning members.
Fig, 14 illustrates the placement of channel-forming rods/wires between two channel positioning members after the channel-forming rods/wires have been bundled together with a bundling member (35).

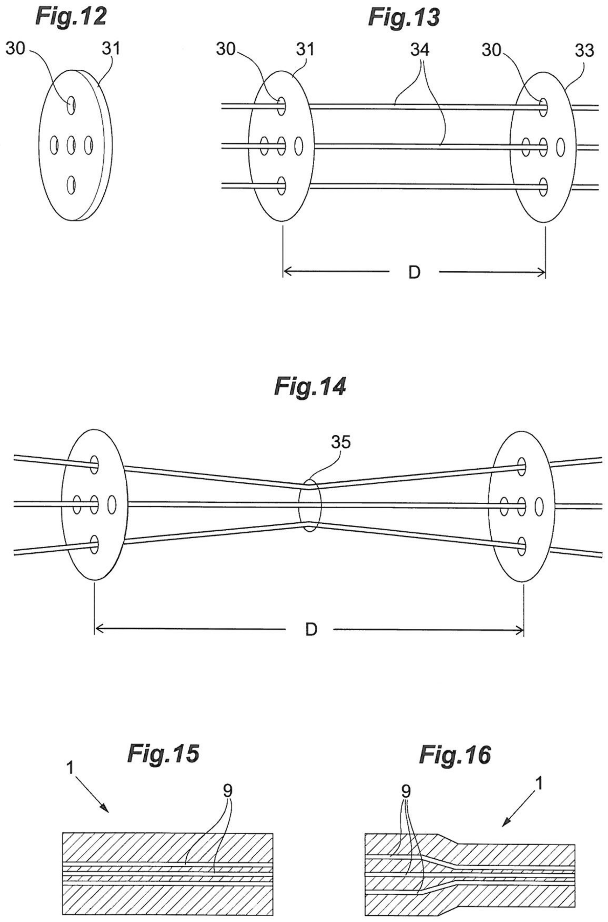

Fig. 15 illustrates a proto microfilament guiding structure of a transient material capable of swelling in liquid.
Fig 16 illustrates the shape of the microfilament guiding structure of fig. 15 after a part of the proto guiding stricture has been allowed to swell.

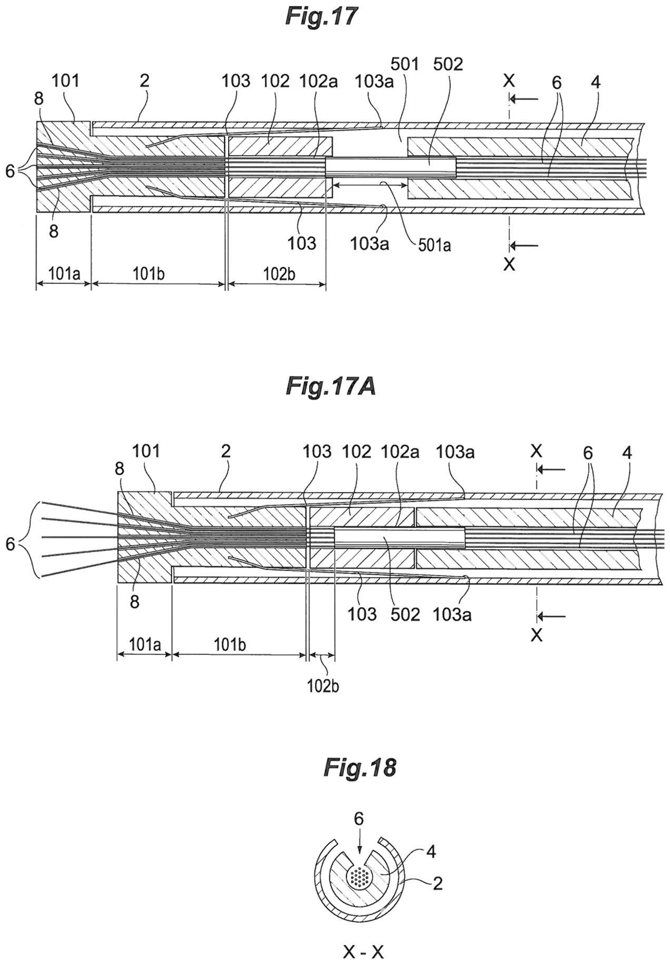

Fig. 17 illustrates a cross-sectional view of embodiment Δ of the microfilament guiding structure disposed in the elongated hollow guiding member together with a separate hollow supporting guide.
Fig. 17A illustrates a cross-sectional view of embodiment Δ of the microfilament guiding structure disposed in the elongated hollow guiding member together with a separate hollow supporting guide in a state where the microfilaments have been propelled out of the microfilament guiding structure in distal direction.
Fig. 18 illustrates a radial cross section view at XX of the assembly of fig. 17 together with a stereotaxic guide.

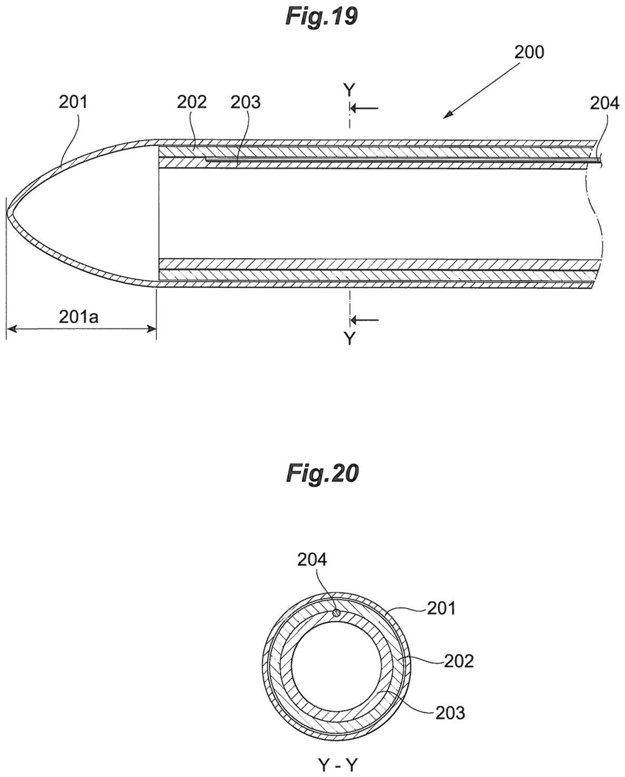

Fig. 19 illustrates a schematic axial cross-section view of the cover. The assembly comprising microfilament guiding structure, pin guiding structure, elongated hollow guiding member is not shown.
Fig. 20 illustrates a radial cross-section view of the cover at YY.

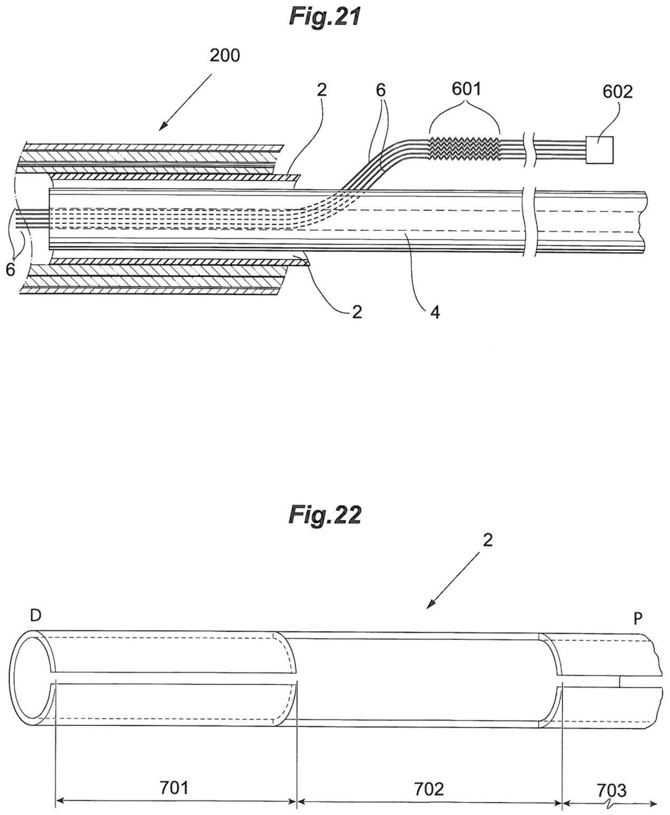

Fig. 21 illustrates an axial view of the assembly with cover, pin, and the microfilaments exiting the assembly in radial direction.
Fig. 22 illustrates a version of the elongated hollow guiding member.

### FURTHER DISCLOSURE OF THE INVENTION

The proto microfilament assembly may be a transient instrument (or comprise several transient items) for the insertion (implantation) and placement of the microfilament assembly within soft tissue. Thus, several parts of the proto microfilament assembly, notably the microfilament guiding structure and optional hollow supporting guide and pin and optionally also the elongated hollow guiding member, gradually disintegrates after insertion. The principle is to implant (position) the proto microfilament assembly and hence the microfilament guiding structure into the soft tissue at a distance from the target and then push (the microfilaments forward out from the microelectrode guide structure so as to be inserted in the target soft tissue. Thereafter parts of the proto microfilament assembly are disintegrated and/or dissolved and absorbed by the tissue and/or are removed. If the elongated hollow guiding member and possibly also the rigid pin secured within the pin guiding structure is degradable the need to extract parts of the proto microfilament assembly is reduced or effectively omitted.

The proto microfilament assembly is indented to be introduced into soft mammalian tissue. The distal region of the proto microfilament assembly is the region which first enters into the soft tissue. Thus, the distal region/section/end of a proto microfilament is usually the region penetrating deepest into soft tissue until the microfilaments have been propelled distally. Concurrently, the proximal region of a proto microfilament is usually closer to the boundary of the mammalian and the environment. Proximal regions of a proto microfilament assembly may also lie outside any mammalian tissue (e.g. the proximal parts of the individual microelectrodes). The designations distal and proximal are also used to spatially designate structures/members/guides having an axial extension. Thus, the distances between at least some of the channels of the microfilament guiding structure are further apart in the distal region than in a proximal region..

It is preferred that the proto microfilament assembly holds together during insertion and a certain amount of time after positioning, thus allowing the flexible microfilaments to be pushed out from the proto microfilament assembly (i.e. from the microfilament guiding structure) a certain distance along predetermined track lines given by the channels of the microfilament guiding structure. The length by which the microfilaments can be pushed out without bending away from intended track line depends on the flexibility of the microfilaments, the insertion speed and tissue resistance and internal tensions of the material of the filament. Since the tissue resistance depends on the insertion speed a higher insertion speed requires stiffer microfilaments.

The proto microfilament assembly is the entity/instrument which is inserted into soft tissue. The proto microfilament assembly should be configured to enable the introduction into soft tissue or into a channel of biocompatible material provided into soft tissue as disclosed by WO 2016/032384 A1. Hence, the proto microfilament assembly should exhibit design features enabling the introduction (insertion) into soft tissue. The proto microfilament assembly has an elongated, oblong axial extent and should also exhibit the stiffness characteristics to enable precise positioning of the distal end of the proto microfilament assembly, i.e. the microfilament guiding structure.

Typically, the cross-section of the proto microfilament assembly is rotationally symmetric, and most preferred are elliptical which includes circular cross-section. The radial extension, diameter, of the proto microfilament assembly is typically under about 2.5 mm, more preferably under about 2.0 mm. The radial extension of the proto microfilament assembly is preferably from about 0.35 mm up to about 2.5 mm, preferably from about 0.5 mm up to about 2.0 mm. Should the cross-section of a proto microfilament assembly be approximately circular, then the diameter would be under about 2.5 mm, more preferably under about 2.0 mm. Several construction parts, such as the microfilament guiding structure and the rigid pin, of the proto microfilament assembly are accommodated by the elongated hollow guiding member. The axial length of the elongated hollow guiding member is typically between about 20 mm and about 250 mm, but can be longer.

It is further beneficial that the proto microfilament assembly exhibits a structural integrity that the assembly can be precisely positioned within soft tissue. The positioning of the assembly is preferably implemented by steadily inserting the assembly deeper into soft tissue (in a distal direction) until a specific position has been reached. The proto microfilament assembly is suitably inserted into soft tissue by application of a stereotactic instrument. For insertion of proto microfilament assembly into animal soft tissue, such as brain tissue, stereotactic instruments such as David Kopf Instruments are practical. High precision stereotactic instruments for human use are also available, such as a Lexell stereotactic frame.

The proto microfilament assembly exhibits the microfilament guiding structure at its distal end. The microfilament guiding structure is accommodated at the distal region of the elongated hollow guiding member. The microfilament guiding structure can have different shapes of the cross-section. Preferably, the microfilament guiding structure should exhibit a cross-section which can be efficiently accommodated in the distal region of the elongated hollow guiding member. The microfilament guiding structure comprises at least two filament guiding channels, the channels providing uninterrupted communication between the distal and proximal sides of the microfilament guiding structure, i.e. the provision of a continuous lumen through the structure. Each guiding channel of the microfilament guiding structure accommodates one microfilament. All microfilaments may be of the same type, alternatively the filament guiding structure may accommodate a variety of microfilament types.

The channels of the microfilament guiding structure are configured to properly guide the microfilaments when introduced into/through the microelectrode (microfilament) guiding structure. The diameter of the channels is suitably adjusted with respect to the diameter of the microfilaments. It is advisable that the diameter of a channel (inner diameter of a channel) is bigger that the diameter of a microfilament (i.e. bigger that the biggest diameter of the section of a microfilament positioned in the channel). It is preferred that the diameter and length of the channel is of a size facilitating the precise guidance of the microfilament within the channel. Preferably, the inner diameter of the channel is not more than twice the size of the diameter of the microfilament. Preferably, the inner diameter of the channel is from about 105% up to about 200% of the diameter of the microfilament, preferably from about 110%, from about 120%, from about 130% up to about 150%, up to about 160%, up to about 170% , up to about 180%, up to about 190% of the extension of the diameter of the microfilament. Any one of the lower ranges may be combined with any one of the upper ranges.

Thus, the microfilament guiding structure may accommodate electrically conductive microelectrodes, hollow filaments for the transportation of a variety of liquids optionally comprising agents with biological effects and filaments comprising additional sensing and stimulating means or optical fibers. The microfilament guiding structure may accommodate any number of channels from 2 to up to several hundred channels. A useful number of channels is between about 5 to about 250, between about 8 up to about 100, between about 10 up to about 80. The diameter of a channel should enable the axial movement of a microfilament. The diameter of a channel of an un-degraded microfilament guiding structure should be approximately equal to or greater than the diameter of the microfilament. The diameter of the channels is suitably from about 5 to about 25 % greater that the diameter of the microfilament. The diameter of the channels may range between about 5 µm up to about 200 µm, suitably from about 10 µm up to about 120 µm. Different channels may have different diameters. For example, the central most channel may be wider than the others.

At least some channels may have the spatial configuration within the microfilament guiding structure that the distance of the distal exit of a channel to the central axis of the microfilament guiding structure is longer than the distance of the proximal exit of the same channel to the central axis.

According to an embodiment at least some of the guiding channels of the microfilament guiding structure have a spatial (three dimensional) configuration that said channels have an angular spatial arrangement with an acute angle α from about 2 deg up to about 40 deg, where the angle α of a channel is given by the acute angle α of a right angled triangle with sides defined by the straight line between the center of the proximal and distal exit of a channel, the straight line parallel to the central axis of the microfilament guiding structure and intersecting the center of the proximal channel exit and the straight line perpendicular to the central axis of the filament guiding structure intersecting the center of the distal exit if the channel and angle α being the angle between the two longest sides. The angle α is suitably in the range of from about 1 up to about 30 deg, such as from about 2 up to 20, such as from 1 to 10.

According to an embodiment, the microfilament guiding structure comprises a distal part positioned distal to the elongated hollow guiding member in axial direction. This distal part positioned distal to the elongated hollow guiding member may be referred to as the ultimate distal section of the microfilament guiding structure. This ultimate distal section may also be the distal section of a discrete distal sub-unit of the microfilament guiding structure. If the microfilament guiding structure comprises two distinct structures they are referred to as distal and proximal microfilament guiding sub-structure. This ultimate distal section of a microfilament guiding structure, or preferably distal section of the distal microfilament guiding sub-structure, incorporates a spatial configuration of individual channels enabling the radial distance of the individual microfilaments to increase as the microfilaments are moved into the soft tissue in distal direction. The ultimate distal section of a microfilament guiding structure, or the distal section of the distal microfilament guiding sub-structure, or distal part of the microfilament guiding structure, suitably has a diameter similar to the outer diameter of the elongated hollow guiding member. It may also have a concave shape such that the distalmost part has the shape of cone or dome. In this case the referred diameter is the diameter of the proximal part of the cone or dome. The diameter of the proximal section of the distal microfilament guiding sub-structure, or the proximal section of a microfilament guiding structure, is such that this proximal section can be accommodated inside the most distal part of the elongated hollow guiding member. Similar diameter in this context means a diameter deviating less than 10%, preferably less than 5% of the outer diameter of the elongated hollow guiding member.

In some embodiment, the ultimate distal section of the microfilament guiding structure may be equivalent to the distal part of the microfilament guiding structure, alternatively, the ultimate distal section of the microfilament guiding structure may be a distal section of the distal microfilament guiding sub-structure.

The radial extension of the microfilament guiding structure is in the range of from about 250 µm up to about 2.5 mm. The axial extent of the microfilament guiding structure is in the range of from about 0.5 mm up to 25 mm, but can be even longer when implemented into deeply located targets.

The channels which are fanning out in radial direction and characterized by any of the above embodiments may have different longitudinal shapes between the distal and proximal exit. Preferably, these channels have a longitudinal shape facilitating the axial movement of the microfilaments in distal direction. The channels fanning out radially may have a continuous curvature such as a concave curvature between the distal and proximal exit. Also, the channels may have a configuration such that the radial deviation is fully provided proximally in the distal section of a microfilament guiding structure, or fully provided in the proximal section of the distal microfilament guiding structure, and, that the channels are essentially linear in the ultimate distal section of the microfilament guiding structure, or that the channels are essentially linear in the distal section of the distal microfilament guiding sub-structure. According to an embodiment, the channels in the distal part of the microfilament guiding structure (comprised in embodiment Δ) are preferably essentially linear and radially fanning out while the respective bends of each channel are provided for in the distal region of the proximal part of the microfilament guiding structure.

According to an embodiment, the microfilament guiding structure as well as the optional pin guiding structure and hollow supporting guide (which will be characterized hereinafter) comprise transient materials including any material or material combination which disintegrate and/or dissolve when contacted by mammalian tissue fluids. Suitably materials are described hereinafter.

According to another embodiment, the distal microfilament guiding sub-structure, or distal microfilament guiding structure of embodiment Δ, may comprise a flexible material which does not dissolve and/or disintegrate over time of body fluids. Such flexible material may be selected from flexible biocompatible polymers including silicone-type polymers and parlylene-based polymers. In this embodiment, the proximal microfilament guiding sub-structure comprises transient materials including any material or material combination which disintegrate and/or dissolve when contacted by mammalian tissue fluids.

The channels may be lined with a material differing in characteristics as to the bulk material, yet gradually disintegrating and/or dissolving when contacted by mammalian tissue fluids.

The microfilament guiding structure in some of the embodiments may be characterized as comprising a distal, ultimate distal and proximal sections, the distal section (and ultimate distal section if applicable) accommodating the channels which are fanning out radially and the proximal section characterized as the section comprising from one channel accommodating all microfilaments up to a number of channels equaling the number of channels in the distal section.. If the proximal section comprises two or more channels they may be essentially aligned parallel to the central axis. The transition from proximal to distal section may be characterized as the approximate location where the at least some of the channels begin to deviate in radial direction. According to embodiment Δ, the axial realms of he microfilament guiding structure is referred to as distal part and proximal part.

According to an embodiment the proximal section of the microfilament guiding structure may be a discrete structure then referred to as a proximal microfilament sub-structure. If the proximal section of the filament guiding structure is a discrete structure it is referred to as a proximal microfilament guiding sub-structure herein. Conversely, if the filament guiding structure comprises two discrete structures, one discrete structure may be referred to as a distal microfilament guiding sub-structure and the other a proximal microfilament guiding sub-structure. As elaborated, the distal microfilament sub-structure may comprise a distal section and a proximal section. This distal section of the distal microfilament guiding sub-structure may also be referred to as ultimate distal section of the distal microfilament guiding structure. In the context of the microfilament guiding structure a section denotes a certain part of a microfilament guiding structure or a certain part of a microfilament guiding sub-structure.

All characteristics related to a proximal section of the filament guiding structure may also be relevant to a discrete proximal microfilament guiding sub-structure.

The combined cross-section area of the channel or channels of the proximal region of the filament guiding structuremust be larger than the combined cross-section area of all microfilaments passing through the proximal region of the microfilament guiding structure.

A further characteristic of the proximal region (or part) of the microfilament guiding structure is that the channel or channels preferably are centrally positioned and if two or more channels are present such channels are essentially parallel and their individual axes essentially coincide with the main axis of the distal region of the microfilament guiding structure.

According to an embodiment, the microfilament guiding structure comprises channels (and optionally a channel or channels coinciding with the central axis or being parallel to the central axis) with a spatial arrangement characterized that the distance of the distal exit of a channel to the central axis of the microfilament guiding structure is longer than the distance of the proximal exit of the same channel to the central axis and that the longitudinal shape of the channels are essentially defined by a straight line between the distal and proximal exit of the channel. (A microfilament guiding structure with a linear arrangement of the channels between distal and proximal exits does not exhibit markedly distal or proximal sections.)

The microfilament guiding structure is suitably rotationally symmetric. As iterated above the microfilament guiding structure can be characterized as comprising at least two regions, a distal and proximal region.

According to an embodiment, one parameter which may distinguish the distal and proximal regions of the microfilament guiding structure is the area of the distal and proximal cross-section. The cross-section area of the proximal region of the microfilament guiding structure is bigger than the cross-section area of the distal region.

According to a further embodiment the distal region of the microfilament guiding structure has a cross-section area which is greater than the cross-section area of the proximal region of the microfilament guiding structure. The cross-section areas are suitably rotationally symmetric, preferably circular.

According to a further embodiment, related to a microfilament guiding structure comprising two discrete structures, a distal microfilament guiding sub-structure and a proximal microfilament guiding sub-structure and the distal microfilament guiding sub-structure comprising a distal and a proximal section, the cross-section area of the distal section of the distal microfilament guiding sub-structure is larger than the cross-section area of the proximal section of the distal microfilament guiding sub-structure. Moreover, the cross-section area of the proximal section of the distal microfilament guiding sub-structure is suitably comparable to the cross-section area of the proximal microfilament guiding sub-structure. All cross-sections of the distal and proximal substructures are preferably rotationally symmetric and suitably rotationally symmetric cross-sections are preferred exhibiting a smaller area, a suitable cross-section is elliptical or circular. The microfilament guiding structure and the elongated hollow guiding member are configured that the combined assembly provides that the microfilaments can be moved independently of the microfilament guiding structure. More precisely, the combined assembly provides that the microfilaments can be moved without significant displacement of the microfilament guiding structure.

In the embodiments featuring a microfilament guiding structure comprising a section distal to the distal end of the elongated hollow guiding member the microfilament guiding structure or at least the distal microfilament guiding sub-structure is secured to the elongated hollow guiding member such that the microfilament guiding structure or the distal microfilament guiding sub-structure does not essentially move in a radial or axial (distal-axial) direction when the microfilaments are pushed in distal direction out of the microfilament guiding structure. One option to attach the microfilament guiding structure to the elongated hollow guiding member is by way of slide-inhibiting filaments comprised in the microfilament guiding structure and attached to the hollow guiding member. The attachment of the slide-inhibiting filaments to the hollow guiding member can be accomplished by a glue that preferably dissolves over time in body fluids. The slide-inhibiting filaments may be inserted into the microfilament guiding structure during formation of the microfilament guiding structure, e.g. during casting. If the slide-inhibiting filaments are attached to the microfilament guiding structure comprising a material which dissolves and/or disintegrates in body fluids the hollow guiding member can be removed from the soft tissue once the dissolvable and/or degradable material of the microfilament guiding structure has.

According to an embodiment, a first part of the distal region of the filament guiding structure may have a cross-section which has an essentially uniform radial extension; radial extension distal region: Rd1. Preferably this first part of the distal region of the microfilament guiding structure has an elliptically or circular cross-section. A second part of the distal region of the microfilament guiding structure may exhibit a cross-section comprising a radial protrusion. According to an embodiment, the cross-section along the distal region of the microfilament guiding structure may have two different areas/cross-sections, e.g. one first area/cross-section (first part of distal region) which is circular with a radial extension (Rd1) with radius rd1 and a second area/cross-section (second part of distal region) with radius rd2 exhibiting two different radial extensions (Rd1 and Rd2) wherein these extensions occupy different angular sections. This may also be expressed as that the distal region has two different radial extensions. For the case that the radial extensions has a circular shape, the axial extension is equivalent to the diameter (also applicable to the proximal region of the microfilament guiding structure). The radial protrusion of the cross-section of the distal region of the microfilament guiding structure (typically having a radial extension Rd2) may have an angular extension from a few deg up to about a value which is smaller than the angular extension of the wide radial extension of the cross-section of the proximal region of the microfilament guiding structure. Typically, the angular extension of the radial protrusion of the cross-section of the distal region is between about 5 deg up to about 90 deg, preferably from about 5 deg up to 45 deg, such as from about 5 deg up to about 15 deg. According to an embodiment, the radial extension of the protrusion of the distal region of the guiding system Rd2 is equal to Rwp (as defined below). According to a further embodiment, the radial extension of the distal region having an essentially uniform radial extension is equal to Rnp.

The proximal region of the microfilament guiding structure preferably has a uniform cross-section along the entire the proximal region. Alternatively, the cross-section of the proximal region of the microfilament guiding structure has two angular sections exhibiting two different radial extensions, a wide radial (proximal) extension Rwp with radius rwp and a narrow radial (proximal) extension Rnp with radius rnp. The radial extensions of the proximal cross-section are suitably elliptical or circular. It is also preferred that the combined angular extensions of the two different radial extensions of the cross-section of the proximal region of the microfilament guiding structure is 360 deg. The angular extension of the wide radial extension is suitably from about 90 deg up to about 270 deg, preferably from about 90 deg up to about 180 deg. An angular extension of up to about 180 deg may facilitate the assembly of the proto microfilament assembly, and more specifically the assembly of the microfilament guiding structure and the elongated hollow guiding member.

The microfilaments may be selected from electrically conductive microelectrodes, hollow filaments for the distribution of biologically potent agents and filaments comprising additional sensing means.

It is favorable if the microfilament is configured to facilitate the guidance through the microelectrode guiding structure. A parameter which can facilitate the introduction though the channels of the microfilament guiding structure is the shape and difference of flexibility in axial direction of the microfilament. The thickness, diameter of the microfilament may vary along the microfilament. The microfilament may be configured that the diameter of the microfilament gradually increases in distal direction, alternatively gradually decreases in distal direction. Furthermore, the physical properties, such as the degree of flexibility, of the microfilament may vary along its length. Also, the greatest radial extension of a microfilament should at least not exceed the diameter of the guiding channel. The rigidity of the electrode may be augmented along the distal portion of the microfilament. Rigidity enhancement may be accomplished by an increase of the diameter and/or change in properties of the microelectrode material and/or by the addition of rigidity providing structural elements.

According to a further aspect, the distal end of the microfilament may feature a distal end member which serve to reduce or prohibit soft tissue micro-haemorrhage such as disclosed in WO 2017/095288. The member of the very distal end of a microfilament fully encloses the distal end to form a blunt distal bulge. The bulge has typically a radial extension larger than the radial extension of the distal end of the microfilament.

According to an aspect, the microfilament may be coated by gelatin. Also, the thickness of the insulation material around a microfilament may be different along the length and provisioning a modulation of the flexibility of a microfilament along the length.

Electrically conductive microelectrodes preferably comprise an electrode body preferably comprising a noble metal or an alloy of noble metals or comprising noble metals such as gold, silver, platinum, iridium, but other biologically acceptable metals such as stainless steel and tantalum can also be used as well as gold plated copper. The metallic surface of the electrode body can be modified by applying a layer of another metal or metal alloy or a layer comprising or consisting of an electrically conducting non-metallic material. Alternatively, the electrode body may consist of or comprise an electrically conducting polymer (e.g. poly(3,4-ethylenedioxythiophene) (PEDOT)) and/or electrically conducting carbon such as graphite or graphene optionally in form of nanotubes. Alternatively, the electrode body can be made of a core of nonconductive polymer material coated with a metal, in particular a noble metal. The portions of the electrode body lacking insulation may be advantageously provided with surface enlarging elements or structures such as a roughened surface, forests of conducting nanowires, for instance carbon nanowires, or be porous. Surface enlarging structures of this kind will reduce the impedance of the electrode body. A part of the electrode body is typically encased with an insulating material comprising or consisting of polymer materials, in particular polymers selected from the group consisting of Parylene, polytetrafluoroethylene (Teflon), polyurethane and silicone. Other suitable insulating materials include biocompatible polymer materials, inorganic materials such as hafnium oxide.

It is preferred that the electrically conductive microelectrodes are oblong/elongated. The term 'microelectrode' is meant also to include an insulating layer except regions in electrical contact with tissue. The radial extension of a microelectrode (including insulation layer) can be from about a few microns (µm), e.g. from about 2 µm up to about 200 µm, suitably from about 5 µm up to about 80 µm. The radial extension of a microelectrode can be from 2, 4, 6, 8, 10, 15, 20 µm and up to 50, 60, 70, 80, 90, 100, 110, 120, 150, 180 µm. Any one of the lower levels may be combined with any one of the higher levers. A radial extension between about 10 µm to about 60 µm is particularly suitable. The microelectrodes are preferably rotationally symmetric, preferably exhibiting an elliptical or circular cross-section. The length of a microelectrode is partly governed by the location of the soft tissue to be monitored and/or activated and specifically the depth of the soft tissue to be monitored. The microelectrodes should have a length enabling the electric communication from the location inside soft tissue to a location externally to the soft tissue to be examined/monitored, such as external of a mammalian body or electronics implanted externally to the soft tissue. The length of a microelectrode may range from a few millimeters (mm) up to several centimeters, such as from a few mm up to 50 mm or even up to 250 mm or more

The proto microfilament assembly provides for the insertion of a cluster of flexible microfilaments into soft tissue from the microfilament guiding structure. Hence, once the proto microfilament assembly is positioned within soft tissue the cluster of flexible microfilaments accommodated inside the guiding channels of the microfilament guiding structure are carefully pushed out of the channels into the soft tissue. The movement of the microfilament cluster is achieved by the movement of the pin in distal direction to which it is attached. The movement of the pin is possible by exerting an axial force to the rigid pin which, at least distally, is accommodated within the hollow guide member..

The flexibility of the microfilament which to an extent is provided by its limited radial extent reduces the irritation of the soft tissue during insertion. Furthermore, the flexibility of the microfilament provides for compliance to the soft tissue once the microfilament has been properly positioned. The flexibility of the microfilament inducing compliance reduces mechanical forces between the microfilament and the immediate surrounding tissue thereby reducing the formation of encapsulating tissue between microfilament and cells to be studied in the soft tissue. Even if the microfilaments are highly flexible the proto microfilament assembly provides the conditions for enabling the insertion of the flexible microfilaments into soft tissue provided that the flexible microfilaments do not have to traverse tougher structures such as meningues as described above.

A feature of the proto microfilament assembly is the movement of the microfilaments into the soft tissue once the assembly has been properly positioned. One function of the pin is to exert an axial force in distal direction on the microfilaments. The microfilament guiding structure being essentially spatially fixated by the hollow guiding member, the proto microfilament assembly should exhibit a gap between the microfilament guiding structure and the pin. The axial extension of the gap depends on various factors including the flexibility of the microfilaments. The maximum extension of the microfilaments from the microfilament guiding structure is highly correlated to the extension of the gap. Stiffer microfilaments may be extended further into soft tissue than less stiff (more flexible microfilaments). Thus, the stiffness or flexibility of the microfilaments may have an influence on the placement of the microfilament guiding structure and pin within the elongated hollow guiding member. The gap (distance) between the filament guiding structure, or the hollow supporting guide, and the pin is typically from about 500 µm up to several millimeters such as up to about 20 mm but can be longer, such as up to 60mm or more in case a hollow guide structure is placed between the filament guiding structure and the pin.

According to an embodiment the pin is attached to a pin guiding structure. Preferably, the pin is suitably centrally disposed within the pin guiding structure. Pin and rod is used interchangeable. The pin/rod is preferably transiently attached to the pin guiding structure. By transiently attached should be understood that the rod is secured to the pin guiding structure in the proto state of the microfilament assembly. Once the proto microfilament assembly is disposed in soft tissue the pin guiding structure gradually disintegrates and/or dissolves. The pin guiding structure may preferably comprise or consist of any of the materials relevant to the microfilament guiding structure disclosed herein. The rod preferably protrudes from the pin guiding structure in proximal direction and can be attached to a micromanipulator in its proximal end. The microfilaments are preferably positioned between the pin guiding structure and the rod. The rod, microfilaments and pin guiding structure may suitably be held together by way of glues on a carbohydrate or protein bases such as alkylated and/or carboxylated cellulosa derivatives, amylose, and gelatin, but can also be of a different nature, such as polyvinyl alcohol, polyvinylpyrrolidone, and alkali salts of polyacrylic acid. The glue should preferably exhibit the qualities of gradually dissolving and/or disintegraing when contacted by soft tissue (i.e. aqueous mammalian tissue fluids) while providing binding properties when in dry state.

The microfilament guiding structure may e.g. contain 100 channels for the accommodation of 100 individual microfilaments. The channels of the microfilament guiding structure have a spatial configuration such that the axes (centerline) of each channel increase in distal direction. Once the microfilament guiding structure is formed 100 individual microfilaments are inserted into each of the 100 channels eventually providing a proto microfilament assembly containing 100 individual microfilaments. The spatial reach of all microfilaments of the cluster is to a certain extent dependent on the diameter of the proto microfilament assembly governing the number of individual microfilaments which can be accommodated. The diameter of the proto microfilament assembly is defined by the type of soft tissue. For nervous tissue the proto microfilament assembly may e.g. have a diameter of 2 mm. This proto microfilament assembly with a diameter of 2 mm may comprise a microfilament guiding structure containing 100 channels and 100 microelectrodes, one microelectrode per channel. The channels of the microfilament guiding structure are configured that at any given point along the main axis of the microfilament guiding structure the axes of the channels gradually increase in distal direction. In this configuration the proto microfilament assembly provide a cluster of 100 evenly distributed microfilaments precisely positioned within soft mammalian tissue, e.g. nervous tissue. This specific example of proto microfilament assembly can create a cluster of microfilaments in soft tissue with an average distance between individual microfilaments of around 100 µm to 1 mm separation.

A further part of the proto microfilament assembly is the elongated hollow guiding member. The elongated hollow guiding member is of a biocompatible material with sufficient structural rigidity to provide a proto microfilament assembly with sufficient structural rigidity that the distal end of the proto microfilament assembly, i.e. the microfilament guiding structure can be precisely positioned within soft tissue by application of e.g. a stereotactic instrument.

The elongated hollow guiding member suitably comprise two distinctive regions, a distal and a proximal region. The elongated hollow guiding member needs to have a spatial configuration suitable to accommodate the microfilament guiding structure. As the pin is also accommodated in the elongated hollow guiding member and intended to slide in distal direction with respect to the elongated hollow guiding member it is preferred that the elongated hollow guiding member exhibits an elliptical cross-section or an annular circular cross-section (annular may imply a circular shape). However, the distal region of the elongated hollow guiding member may exhibit internal design elements preventing the microfilament guiding structure to move axially with respect to the elongated hollow guiding member. According to an embodiment, the elongated hollow guiding member comprises a lateral partition along the entire length of the elongated hollow guiding member. According to a further embodiment the elongated hollow guiding member exhibits an annular cross-section with an inner diameter Di and an outer diameter Do. The lateral partition of the distal portion of the elongated hollow guiding member is preferably smaller than the lateral partition of the proximal portion of the elongated hollow guiding member. According to a further embodiment, the lateral partition of the annular cross-section of the proximal region of the elongated hollow guiding member expressed as angular extension is preferably between about 90 deg up to about 270 deg. The lateral partition of the annular cross-section of the distal region of the elongated hollow guiding member has preferably an angular extension capable of accommodating the distal region of the microfilament guiding structure.

According to an embodiment of the wide radial (proximal) extension Rwp of the proximal cross-section of the microfilament guiding structure is preferably between about -10% up to the outer diameter Do of the elongated hollow guiding member. Furthermore, the radial extension of the protrusion of cross-section of the distal region of the microfilament guiding structure is preferably between about -10% up to the outer diameter Do of the elongated hollow guiding member.

According to an embodiment the length of the distal region of the microfilament guiding structure is between about -10% up to about 10% of the length of the distal region of the elongated hollow guiding member, preferably between about -5 % up to about 5%, suitably between about -3% up to about 3%, such as between about -1% up to about 1%.

The elongated hollow guiding member should be biologically compatible and, according to an aspect, not to be susceptible to degradation by mammalian tissue. The elongated hollow guiding member may be constructed of any biocompatible material providing sufficient rigidity to the proto microfilament assembly. Furthermore, it is preferred that the material has the properties to allow the pin to slide. Either the material of the elongated hollow guiding member provides sliding properties per se, alternatively, the elongated hollow guiding member comprises a surface layer facilitating the pin or pin guiding structure to slide. Useful materials of the elongated hollow guiding member are metals, polymers or any one of the materials for the transitional microfilament guiding structure or pin guiding structure or hollow supporting guide. If transient materials are used for the elongated hollow guiding member, i.e. materials which disintegrates and/or dissolves in soft tissue, they must provide significant structural rigidity to the elongated hollow guiding member such that the proto microfilament assembly can be precisely spatially positioned in soft tissue. Hence, the transient materials should typically provide sufficient rigidity that the proto microfilament assembly is successfully disposed within the soft tissue before dissolution and/or disintegration has reached a point where successful disposition of the microelectrode is prohibited. Polymeric materials may be reinforced by fibers. Suitable metals for the elongated hollow guiding member are surgical grade stainless steels such as austenitic SAE 316 and martensitic SAE 440, SEA 420 and 17-4 stainless steels. A preferred material for rigid elongated hollow guiding member is carbon fiber or surgical grade stainless steel.

Any materials of the proto microfilament assembly should be biocompatible.

The microfilament guiding structure, pin guiding structure and optionally the elongated hollow guiding member may comprise or consist of materials, specifically materials forming gels when contacted by mammalian fluids, i.e. gel-forming materials, or gels, selected from the group consisting of low molecular carbohydrates, proteinaceous materials or mixtures thereof.

Gelatin from various animal sources including recombinant gelatin is a preferred material of the microfilament guiding structure. Gelatin may be sources from fish and mammals. The rigidity and degradation of gelatin may be modulated by cross-linking. Examples of gelatin cross-linking agents are glyceraldehyde, bis(vinylsulfonyl)methane and 1-ethyl-3(3-dimethylamino-propyl)carbodiimide. Another useful crosslinking method is by UV radiation. The rate of degradation in the body can be controlled by the extent of cross-linking, which in turn can be controlled by the amount of cross-linking agent used or by controlling the exposure to UV radiation used for crosslinking a given amount of gelatin.

Other aqueous biocompatible gels of the invention include carbohydrate gels. Carbohydrate gels useful in the invention include arabinogalactan gel, arabinoxylan gel, galactan gel, galactomannan gel, lichenan gel, xylan gel but also cellulose derivatives such as hydroxymethylpropyl cellulose, and are formed by contact with aqueous media, in particular aqueous body fluid, with a gel forming agent selected from: arabinogalactan, arabinoxylan, galactan, galactomannan, licenan, xylan, hydroxymethyl cellulose and other cellulose derivatives forming gels in contact with aqueous media.

Further aqueous biocompatible gels of the invention include protein gels. Protein gels other than gelatin from animal sources useful in the invention include whey protein gel, soy protein gel, casein gel, which are formed by contact of aqueous media, in particular aqueous body fluid with a gel forming agent selected from whey protein, soy protein, casein.

Useful materials for any of the transient parts of the assembly including the microfilament guiding structure and optional hollow supporting guide and pin guiding stricture include hyaluronic acid in native and cross-linked form; whey protein, soy protein, casein; arabinoxylan; galactan; galactomannan; lichenan; xylan; cellulose derivatives such as hydroxymethylpropyl cellulose; chitosan; gum Arabic; carboxyvinyl polymer; sodium polyacrylate; carboxymethyl cellulose; sodium carboxymethyl cellulose; pullulan; polyvinylpyrrolidone; karaya gum; pectin; xanthane gum; tragacanth; alginic acid; chitin; poly-glycolic acid; poly-lactic acid; co-polymer of poly-glycolic and poly-lactic acid; co-polymer of polylactic acid and polyethylene oxide; polyethylene glycol; polydioxanone; polypropylene fumarate; poly(ethyl glutamate-coglutamic acid); poly(tert-butyloxy carbonylmethyl glutamate); polycaprolactone; poly(caprolactone-co-butylacrylate); poly-hydroxybutyrate and copolymers thereof; poly(D,L-lactide-cocaprolactone);poly(glycolide-co-caprolactone); poly(phosphate ester); poly(amino acid); poly(hydroxybutyrate); polydepsidpeptide; maleic anhydride copolymer; polyphosphazene; polyiminocarbonate; poly[(7.5% dimethyl-trimethylene carbonate)-co-(2.5% trimethlyene carbonate)]; polyethylene oxide; homopolymer of polyvinyl alcohol; hydroxymethyl methacrylate; hydroxyl- or amino-terminated polyethylene glycol; acrylate-based copolymer such as methacrylic acid, methacrylamide; heparan sulfate; RGD peptide; polyethylene oxide; chrondroitin sulfate; YIGSR peptides; keratan sulfate; VEGF biomimetic peptide; perlecan (heparan sulfate proteoglycan 2); Ile-Lys-Val-Ala-Val (IKVAV) containing laminin alpha-1 chain peptide; modified heparin; fragment of fibrin; and their combinations.

According to an embodiment the proto microfilament assembly, in particular the microfilament guiding structure, may enable the administration of pharmacologically active agents. Such pharmacologically active agents may include coagulant, anticoagulant, antibiotic, osmotic pressure adjusting agent, anti-inflammatory agent, nutrient, factor stimulating growth, factor stimulating cell differentiation, hormone. The administration of pharmacologically active agents can be made through a microfilament designed for the administration of pharmacologically active agents. Such a microfilament may for example be disposed in a channel which is essentially straight and positioned essentially along the central axis..

According to yet a further embodiment the proto filament assembly may comprise an outer layer of materials selected from the group consisting of low molecular carbohydrates, proteinaceous materials such as proteoglycans or glycoproteins and mixtures thereof, preferably gelatin.

The present invention also encompasses methods for manufacturing a microfilament guiding structure and a proto microfilament assembly. Flexible rods and and/or wires and/or filaments may be used in the manufacturing. When referring to rod it is implicitly understood that a rod can be replaced by a wire of filament.

The microfilament guiding structure may be manufactured by spatially arranging channel forming flexible rods and and/or wires and/or filaments which will serve as microfilament channel-forming structures once positioned into a mold. The channel forming rods and/or wires may be covered by a material which will form the channel surfaces of the microfilament guiding structure.

After positioning the channel forming rods within a mold a suitable material of any one of the materials presented herein usable as microfilament guiding structure materials are provided in a state enabling casting. After the material has transitioned to a state of structural rigidity the mold is removed and the rods are removed. Gelatin is one preferred material which may be used for manufacturing the microfilament guiding structure. The microfilament guiding structure may be fabricated by providing a solution of dissolved gelatin which is poured into a mold comprising flexible rods/wires serving as microfilament channel-forming structures. After the gelatin has solidified and optionally has dried the mold and channel-forming rods/wires are removed. The gelatin may be cooled to close to 0 °C before the flexible rods are removed. The microfilament guiding structure is suitably freeze-dried in a temperature of below -20 °C.

The diameter of the wires/rods should be greater than the diameter of the microfilaments which are introduced into the channels of the microfilament guiding structure.

A further method for manufacturing the microfilament guiding structure is the arrangement of several wires/rods parallel to each other within a mold (fig. 15). The mold preferably has a tubular shape with a circular cross-section. The wires/rods are suitable centrally disposed within the mold with one rod/wire coinciding with the central axis of the circularly shaped mold. After solidification of the casting material which can be any one of the transient materials identified herein (any material disintegrating and/or dissolving when contacted by soft tissue, tissue fluids) the mold and rods/wires are removed. The formed microfilament guiding structure may be cooled down to near 0 °C before the rods/wires are removed. In a further manufacturing stage the distal region of the microfilament guiding structure is allowed to swell (Fig. 16). The swelling may be achieved by subjecting a distal region of the guiding system to a coldaqueous solution. The swelling of the microfilament guiding structure increases the distance of the axes of the channels in the distal region of the microfilament guiding structure. The obtained microfilament guiding structure comprising channels having a spatial (three dimensional) configuration where the distance of the axes of individual channels increase in distal direction may in a further step be covered by an additional layer of transient material providing a circumference having a constant radial extension in axial direction.

Yet a further method for manufacturing the microfilament guiding structure comprises providing a first and second channel positioning member comprising openings (30). Suitably, but not necessarily, the first (fig. 13: 32) and second (fig. 13: 33) channel positioning member have the openings (3) positioned at the same locations. The first and second channel positioning member are positioned at a suitable distance D to each other. Next, flexible wires/rods (34) are positioned through one opening of each channel positioning member. The wires/rods may be secured at the respective channel positioning members or further away. At a suitable position between the channel positioning members, the wires/rods are bundled together. The bundling may be achieved by a rigid bundling member (fig. 14: 35) or a flexible wire/filament.

Advantageously, the first and second channel positioning members may additionally constitute parts of the mold. The first and second channel positioning members may form the bases of a cylindrical mold. After positioning the channel arrangement, i.e. first and second channel positioning members and wires/rods bundled together between first and second channel positioning members, within a mold a suitable transient material or combination of transient materials is poured/injected into the mold. After solidification of the material the mold is opened, and the wires/rods are removed and a channel comprising structure is formed. Before the structure is separated from the mold, alternatively, after separation from the mold but before the wires are removed, the structure is suitably cooled down to a temperature range below about 10 °C down to above 0 °C or cooler and freeze dried.

In a further step the channel comprising structure is cleaved in at least two parts, preferably perpendicular to the axis of the channel comprising structure whereby two distal microfilament guiding structure are formed.

The microfilaments may be positioned during formation of the microelectrode (microfilament) guiding structure, yet, it is preferred that the filament guiding structure is fabricated before the microfilaments are inserted. The microfilaments may be introduced from either the distal or proximal side of the filament guiding structure.

According to an embodiment applicable to most manufacturing varieties, one microfilament is attached to one rod/wire. The attachment of a microfilament to a rod/wire can be accomplished at any stage in the manufacturing process as long as the result is that a microfilament is simultaneously inserted into a guiding channel when the channel-forming rod/wire is withdrawn from the solidified microfilament guiding structure. At one end of the rod part the rod is chipped away axially and laterally providing a non-circular cross-section. The microfilament is attached laterally to the rod with a non-circular cross-section. Alternatively, one end of a circular rod is configured that a microfilament can be attached laterally, e.g. glue, to the end such that the rod including microfilament do not extend radially beyond the diameter of the rod.

A further embodiment of the invention relates to a method for manufacturing a microfilament guiding structure comprising channels, and microfilaments disposed inside the channels, the method comprising positioning channel forming flexible rods and/or wires in a predefined spatial arrangement, positioning the flexible rods and/or wires having a predefined spatial arrangement in a mold, filling the mold with a casting material liquid form, letting the casting material solidify, forming the channels of the microfilament guiding structure as the flexible rods and/or wires are removed from the microfilament guiding structure and at the same time disposing microfilaments attached to one end of the flexible rods and/or wires inside of the channels. The material for casting is preferably chosen from materials dissolving and/or disintegrating when subjected to tissue fluids such as gelatin. The radial extension of the microfilament guiding structure is preferably below about 2.5 mm. At least part of the microfilaments are microelectrodes having a radial extension up to about 100 µm.

### DISCLOSUTRE OF SPECIFIC EMBODIMENTS

Fig. 17 illustrates the embodiment Δ presented herein. Fig. 17 illustrates a proto microfilament assembly Δ without a cover structure (200). The microfilament guiding structure (101) comprises a distal part (101a) and a proximal part (101b). The distal part of the microfilament guiding structure comprises channels (lumens) (8). The distal ends of the microfilaments (6) are disposed inside the channels (8). The distal channels of the distal part (101a) are essentially linear until reaching the proximal part (101b) of the microfilament guiding structure. In the distal area of the proximal part suitable channel are curved to follow a disposition in the proximal part as being essentially parallel to the central axis of the elongated hollow guiding member (2). All channels in the proximal part of the microfilament guiding structure are disposed centrally. Proximally to the microfilament guiding structure a hollow supporting member (102) is disposed. The hollow supporting guide (102) has a central void (102a) for the disposition of the microfilaments (6). Each microfilament in the hollow supporting guide is individually movable. The diameter of the conduit (102a) is such that the risk of buckling of individual filaments when moved distally is significantly reduced are eliminated. The microfilament guiding structure and the hollow supporting guide are made of gelatin of other materials which disintegrates and/or dissolves upon contact with tissue fluids. Threads (103) anchored into the microfilament guiding structure are attached to the elongated hollow guiding member (103a). These threads (103) may be attached to any part of the elongated hollow guiding member proximally to the microfilament guiding structure. Preferably, the threads are attached to a very proximal part of the microfilament guiding structure. In Fig. 17 two threads (103) are illustrated. The microfilament guiding structure may comprise a plurality of threads. According to a version, the microfilament guiding structure may also be attached to the elongated hollow guiding member by using a degradable glue. Also illustrated in Fig. 17 is a pin (4) comprising the microfilaments. The microfilaments are removably attached to the pin. Suitably, the microfilaments are at least removably attached to the distal section of the pin. The attachment to the pin may be accomplished with any of the degradable glues specified herein. Between the hollow supporting guide (102) and the pin (4) there is a gap (501) with the axial length (501a). The axial distance of the gap governs how far the microfilaments can be propelled into the tissue. For preventing the microfilaments to buckle/bend in the gap (501) the microfilaments are bundled together with a transient material (502) increasing the structural rigidity. This material can be any of the dissolvable or degradable or degradable materials and/or glues specified herein. Preferably, the material providing structural rigidity extends a bit into the conduit of the hollow supporting member (502a). If the material providing structural rigidity does not extend in radial direction into the hollow supporting guide, there is a risk that the individual microfilaments will bend/buckle where they are not supported by the conduit of the hollow supporting guide. Preferably, the length of the hollow supporting guide is longer than the gap (501) between the hollow supporting guide and the pin. Preferably, the length of the hollow supporting guide distally to the bundle of microfilaments comprising material proving structural support (102b) is longer than the gap (501). Fig. 17a illustrates the microfilament assembly after the microfilaments have been propelled into the tissue before dissolution of microfilament guiding structure and hollow supporting guide.

Fig. 17A illustrates a proto microfilament assembly Δ without a cover structure in a state where the microfilaments (6) protrude distally to the microfilament guiding structure (101). Also shown is the pin (4) which is positioned in close proximity to the hollow supporting guide (102).

Fig. 18 illustrates a cross-sectional view of the proto microfilament assemble at X-X. The microfilaments (6) are disposed inside the pin (4) which is disposed inside the elongated hollow guiding member (2) which in turn is disposed in a guide of a stereotaxic instrument further proximally (not shown).

Fig. 19 illustrates a cover structure (200). Not shown in fig. 19 for reasons of clarity is the proto microfilament assembly of fig. Fig. 17. The cover comprises three layers (201, 202. 203). The layer (202) disposed between an outer layer (201) adjacent to tissue (once the assembly is introduced into soft tissue) and the inner layer (203) is a liquid barrier and does not dissolve in tissue liquid. Preferably, this middle layer (202) is made of a thin layer of parylene C but can be made of other polymers. The outer and inner layers (201, 203) comprise any one of the dissolvable, degradable materials disclosed herein. Inner and outer layers may have same properties or different properties with respect to the dissolution in tissue fluid since the outer layer is more exposed to the tissue during implantation it will dissolve faster than the inner layer. Also, different part of the inner and outer layers may have different dissolution profiles. Integrated into the cover is a thread (204). The cover can be partitioned by engaging the thread in lateral direction. Typically, the cover is gradually partitioned (ripped apart) as the assembly and cover (middle layer) is removed from soft tissue by pulling the thread in radial direction. Part of the thread should be positioned radially inside of the middle layer (202) as shown in fig. 19. The thread (204) may partly be integrated in the inner layer (203). It is important that the distal end (201a) of the cover is free from barrier material otherwise it is not possible to propel the microfilaments into tissue.

Fi. 6A illustrates a
Fig. 20 illustrates a cross-sectional view of the cover at Y-Y.

Fig. 21 illustrates an embodiment of the proto microfilament assembly where the bundle of microfilament is not disposed inside the entire axial length of the pin. Instead, at a certain location the microfilaments are deviated laterally (radially) from the pin, elongated hollow guiding member, optional cover and stereotaxic guide. Fig. 21 shows the microfilaments (6), pin (4), the elongated hollow guiding member (2), and cover (202). Further, the microfilaments outside the cover have a 3D configuration (601) accommodating soft tissue movement, in particular the component of movement of soft tissue coinciding with the main axis of the microfilaments disposed in soft tissue. Suitable 3D configurations include zig-zag, meander, coiled spring configurations. Fig. 21 also illustrates a microfilament securing point (602) which can be localized inside soft tissue or skull tissue should the array/cluster of microfilaments be disposed inside neural brain tissue. The securing point may comprise electronic circuitry.

Fig. 22 illustrates an elongated hollow guiding member (2) comprising an axial opening (gap) along the entire length of the guiding member. As illustrated the width of the gap is not uniform. At the distal (701) and proximal sections (703) the width of the gap is smaller than along an axial distance (702) between the distal and proximal sections. Should the microfilaments be deviated laterally as in Fig. 21 such lateral deviation preferably coincides with the middle section (702).

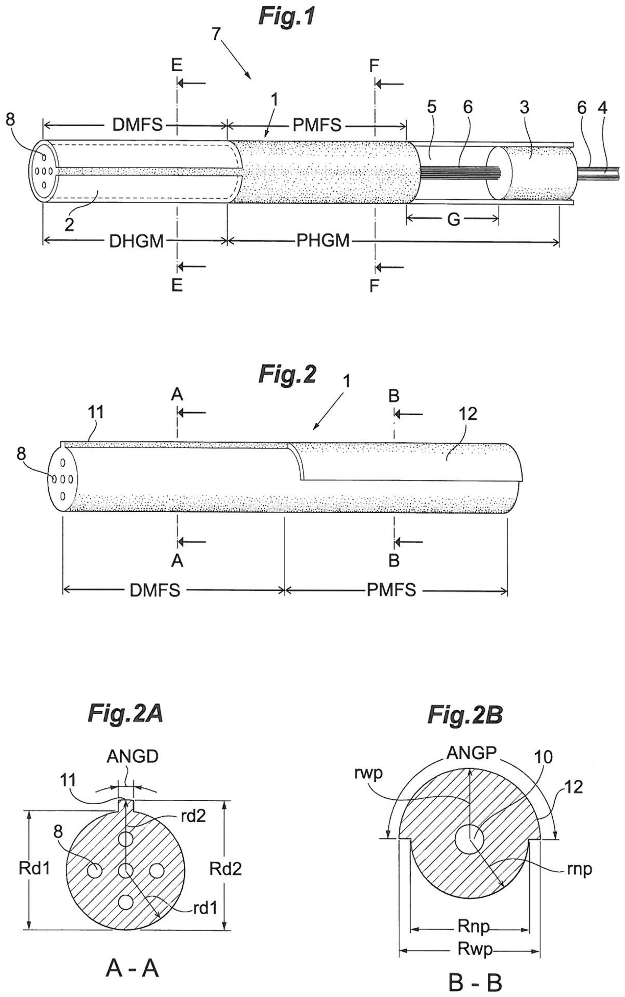

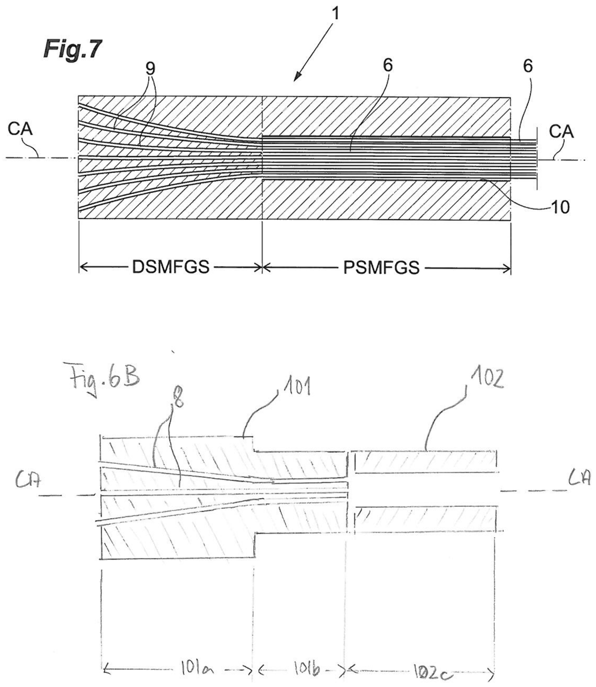

Fig. 1 illustrates a proto microfilament assembly (7) according to the invention. The assembly comprises a microfilament guiding structure (1) accommodated in an elongated hollow guiding member (2). A pin guiding structure (3) is accommodated proximally in the elongated hollow guiding member. A rigid pin (4) is secured to the pin guiding structure (3) and extending from the pin guiding structure in proximal direction. Between the microfilament guiding structure and the pin guiding structure there is a gap (G) in which gap a bundle (5) of microfilaments is indicated. The microfilaments (6) extend through the pin guiding structure and from the pin guiding structure in proximal direction (P). D and P denotes distal and proximal, hence, the D arrow points in distal direction, the P arrow in proximal direction. DMFS and PMFS denote distal and proximal regions of the microfilament guiding structure. DHGM and PHGM denote distal and proximal regions of the elongated hollow guiding member (2). At the distal end of the proto microfilament assembly the microfilament guiding channel exits (8) are indicated. For reasons of clarity the display of microfilaments in the microfilament guiding structure are not shown. The microfilament guiding structure (1) accommodates five microfilament guiding channels. One centrally located channel coincides with central axis of the filament guiding structures. The other four channels may have a spatial configuration within the microfilament guiding structure as schematically illustrated by fig. 6 or fig. 7. Fig. 6 and fig. 7 illustrate lateral cross-sections of microfilament guiding structures (1) with different spatial configurations of the guiding channels (9). For reasons of clarity the channels are depicted by lines. In reality the channels are hollow having a radial extension along their entire length. Fig. 6 and 7 show variants of a microfilament guiding structure (1) where the microfilaments (6) are accommodated in one single channel (10) centrally accommodated within the proximal section of the microfilament guiding structure (PSMFGS). The distal section of the microfilament guiding structure (DSMFGS) denotes the section where at least some of the filament guiding channels (9) gradually begin to radially fan out. During manufacturing the distal and proximal sections of the microfilament guiding structure may be formed separately and subsequently be put together.

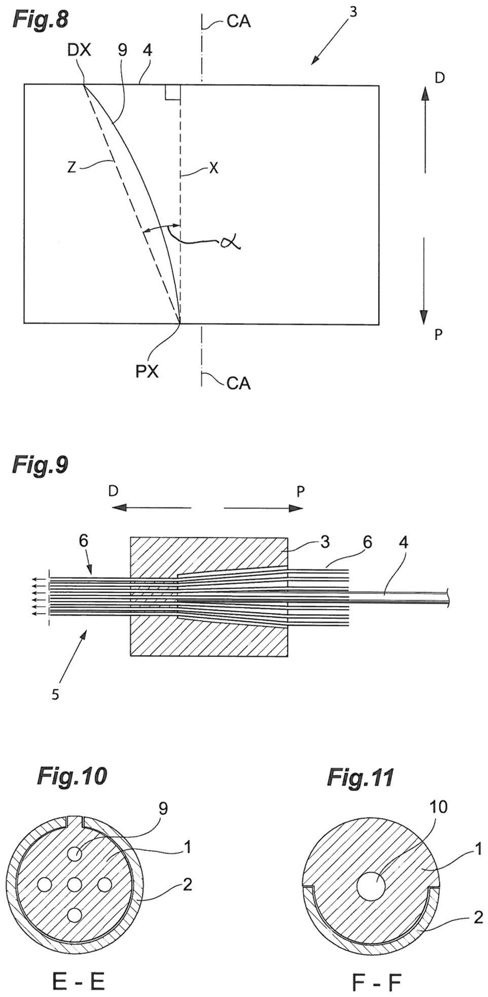

Fig. 2 illustrates a perspective view of a microfilament guiding structure (1) showing the exits of some guiding channels (8). Two distinct regions of microfilament guiding structure are shown: a distal region of the microfilament guiding structure (DMFS) and a proximal region of the microfilament guiding structure (PMFS). Fig. 2a and fig. 2b illustrate cross-sections of the distal region (A-A) and proximal region (B-B) of the microfilament guiding structure. As shown by fig. 2a there is a protrusion (11) of the cross-section of the distal region of the microfilament guiding structure. Also indicated in fig. 2a are guiding channel exits (8). In fig. 2a and 2b ANGD and ANGP denote the angular extension of the protrusion (11) ANGD of the distal region and the angular extension ANGP of the wide radial extension (Rwp) of the proximal cross-section of the proximal region of the filament guiding structure. The sections and region of the microfilament guiding structure may coincide but do not have do to so. Fig. 2a illustrates a cross-section which can be characterized as having two radial extensions, radial extension 1 Rd1 with a radius rd1 and radial extension 2 Rd2 with radius rd2. The angular extension of the protrusion (11) with a radial extension Rd1 and radius rd1 is here shown to be a few degrees. According to fig. 2 the protrusion extends along the entire length of the distal region (DMFS). However, the protrusion may also extend also a part of the length of the distal region. Fig. 10 and fig. 11 illustrate the cross-sections of the proto microfilament assembly of fig. 1 at E-E (distal regions of the filament guiding structure and elongated hollow guiding member) and at F-F (proximal regions of the filament guiding structure and elongated hollow guiding member). In fig. 2 the radial extension (diameter) Rd1 is the same as the radial extension (diameter) of the narrow radial extension Rwp of the proximal cross-section of the proximal region of the filament guiding structure.

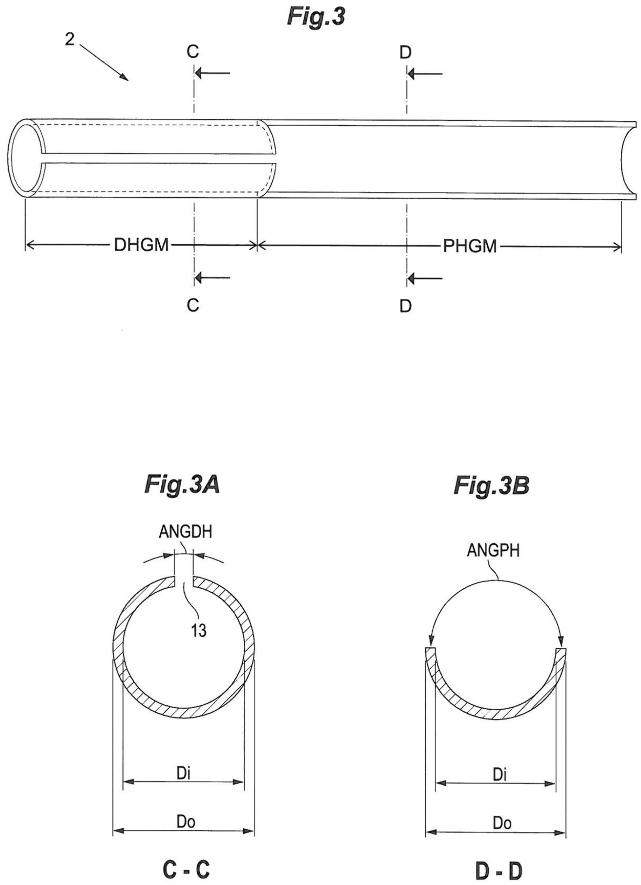

Fig. 3 illustrates a perspective view of an elongated hollow guiding member (2) comprising distal (DHGM) and proximal (PGHM) regions of the elongated hollow guiding member. Fig. 3a and fig. 3b illustrates cross-sectional views of the elongated hollow guiding member at the distal (C-C) and proximal (D-D). The elongated hollow guiding member comprises a lateral partition (13) along the entire length of the elongated hollow guiding member. In this configuration the elongated hollow guiding member has an annular cross-section. Fig. 3a illustrates the cross-section of the distal region of the elongated hollow guiding member. The partition of the distal cross-section (13) is seen at the top. The angular extension of the distal partition (ANGDH) is configured to accommodate the protrusion (11) of the distal region of the filament guiding structure (1). Fig. 3a also shows the inner diameter (D1) and outer diameter (Do) of the cross-section of the distal region. Fig. 3b illustrates the proximal cross-section of the proximal region of the elongated hollow guiding member. The angular extension of the proximal partition is about 180 deg. The inner diameter Di of the elongated hollow guiding member is 1.3 mm and the outer diameter Do is 1.6 mm. The partition of the distal region of the elongated hollow guiding member is about 4 mm. The values indicated are for exemplary purposes and may vary significantly.

Fig. 10 and 11 illustrate cross-sections of the proto microfilament assembly depicted by fig.1 at positions E-E and F-F. Fig. 10 illustrates the distal regions of the filament guiding structure (1) and elongated hollow guiding member (2). Some guiding channels (9) are also indicated. Fig. 11 illustrates the proximal regions of the filament guiding structure (1) and elongated hollow guiding member (2) and the central channel (10) accommodating all microfilaments. For clarity the microfilaments have been omitted. Fig 10 and 11 illustrate an embodiment where the angular extensions of the cross-sections of the filament guiding structure and the elongated hollow guiding member are so adjusted that the combination of filament guiding structure and elongated hollow guiding member provides an elongated entity with a uniform circular cross-section.

Fig. 9 illustrates a lateral cross-sectional view of the pin guiding structure (3). A rigid rod (4) is secured within the pin guiding structure extending in proximal direction P. Distally to the pin guiding structure is a bundle (5) of individual microfilaments (6) presented. Preferably, the microfilaments between the filament guiding structure (1) and pin guiding structure (3) are bundled together with gelatin suitably cross-linked gelatin to provide sufficient stiffness (lateral rigidity) that the axial load in distal direction on the pin guiding structure (3) (effected by the rigid rod) is translated into an axial force component in distal direction on the individual microfilament accommodated in the channels of the filament guiding structure to achieve that the microfilaments are moved out of the channels in distal direction into soft tissue.

Fig. 4 illustrates the axial view of the distal end of the microfilament guiding structure (1) showing the spatial configuration of the exits of the guiding channels (8).

Fig. 5 illustrates a microfilament guiding structure comprising guiding channels arranged linearly along a line from the proximal to the distal end. The channels may also be arranged between the proximal and distal ends along curved lines. A microfilament guiding structure characterized by fig. 5 does not exhibit a section comprising one or more channels arranged parallel to the central axis of the filament guiding structure. A distinction of a filament guiding structure of fig. 5 in a distal and proximal section is not useful.

Fig. 8 illustrates the definition of angle α used to characterize the radial fanning out of the guiding channels of the microfilament guiding structure. In an embodiment the channels have an angular spatial arrangement with an acute angle α of from about 2 deg up to about 40 deg. Fig. 8 illustrates a lateral cross-section of the pin guiding structure (3) indicating the central axis (CA) and a spatial configuration of one guiding channel (9). Angle α is given by the acute angle α of a right angled triangle with sides defined by the straight line between the center of the proximal and distal exit of a channel (line/side Z), the straight line (line/side X) parallel to the central axis of the filament guiding structure and intersecting the center of the proximal channel exit (PX) and the straight line (line/side Y) perpendicular to the central axis of the filament guiding structure intersecting the center of the distal exit (DX) of the channel and angle α being the angle between the two longest sides (lines/sides X and Y).

## Claims

1. A proto microfilament assembly (7) for insertion of microfilaments within soft tissue such as nervous tissue, having a distal region and a proximal region comprising:
- a microfilament guiding structure comprising channels (1, 101),
- microfilaments,
- an elongated hollow guiding member,
- a pin;
wherein microfilaments (6) are disposed in the channels (8) of the microfilament guiding structure (101), the channels (8) providing a continuous lumen through the microfilament guiding structure configured to allow accommodation, and axial movement of the microfilaments, the channels having a diameter and length of a size facilitating the precise guidance of the microfilaments within the channels,
wherein the microfilament guiding structure is of materials which are transient or of materials essentially preserving the configuration over time;
wherein at least in a distal section/part of the microfilament guiding structure the radial distances between at least some of the channels gradually increases in distal direction;
wherein the microfilaments from the microfilament guiding structure (101) extend in proximal direction;
wherein the microfilaments are attached to the pin (4); wherein the pin (4) is located proximally to the microfilament guiding structure (101) and is configured to be slidably disposed inside the elongated hollow guiding member (2);
wherein the proto microfilament assembly is sufficiently stiff to be inserted into a targeted area of soft tissue;
wherein the microfilament guiding structure (101) is essentially spatially fixated by the elongated hollow guiding member (103, 103a);
wherein the microfilaments can be propelled outside of the channels in distal direction by moving the pin in distal direction without essentially moving the elongated hollow guiding member and the microfilament guiding structure.

2. The proto microfilament assembly according to claim 1, wherein the elongated hollow guiding member has an annular cross-section with a lateral opening along at least the distal section and that the microfilaments are removable attached to the pin.

3. The proto microfilament assembly of claim 1 or 2, wherein the rigidity of the microfilaments is enhanced by the addition of rigidity providing structural elements.

4. The proto microfilament assembly of any one of claims 1 to 3, wherein the microfilaments comprise a blunt distal bulge at the very distal end, the bulge preferably having a radial extension larger than the radial extension of the distal end of the microfilament.

5. The proto microfilament assembly according to claim any one of claims 1 to 4, wherein the microfilaments or a part of the microfilament or at least part of the microfilament adjacent and in direct contact with soft tissue, possess a level of internal tensions that prevents the microfilament to substantially deviate from the trajectory provided by the channels of the filament guiding structure during insertion into soft tissue, and having a flexibility sufficient for accommodating movement of soft tissue, such as neural tissue, without significantly affecting the soft tissue.

6. The proto filament assembly according to any one of claims 1 to 5, wherein the diameter of an individual microfilament is from about 5 µm up to about 120 µm, suitably from about 5 µm up to about 70 µm.

7. The proto microfilament assembly of claim 1 or 6, wherein the microfilaments are electrically conductive microelectrodes, the microelectrodes preferably comprising an electrode body, the body comprising noble metals such as gold, silver, platinum, iridium, but other biologically acceptable metals such as stainless steel, tantalum and gold plated copper, or the electrode body consists of or comprise an electrically conducting polymer, electrically conducting carbon, the metallic surface of the electrode body preferably modified by a layer of another metal or metal alloy or a layer comprising or consisting of an electrically conducting non-metallic material; or the body comprises a core of nonconductive polymer material coated with a metal.

8. The proto microfilament assembly of any one of claims 1 to 7, wherein the microfilaments are highly flexible and do not themselves possess sufficient rigidity to be implanted all the way to a target tissue without significantly bending away from their intended track lines.

9. The proto microfilament assembly of any one of claims 1 to 8, wherein the inner diameter of the channels are from about 105% up to about 200% of the diameter of the microfilaments, preferably from about 105% up to about 150%.

10. The proto microfilament assembly according to any one of claims 1 to 9, wherein the microfilament guiding structure comprises a distal part (101a) and a proximal part (101b), the distal part of the microfilament guiding structure disposed distally to the elongated hollow guiding member and the proximal part being disposed inside the elongated hollow guiding member.

11. The proto microfilament assembly according to any one of the preceding claims, wherein the microfilament guiding structure has a radial extension (diameter) from about 250 µm up to about 2 mm, preferably from about 300 µm up to about 2 mm, and preferably an axial extension from about 0.5 mm up to about 25 mm.

12. The proto microfilament assembly according to any one of the preceding claims, wherein the diameter of the channels of the microfilament guiding structure is from about 5 µm up to about 250 µm, suitably from about 10 to about 120 µm.

13. The proto microfilament assembly according to any one of the preceding claims, wherein the microfilament guiding structure gradually disintegrates and/or dissolves when contacted by tissue fluids, and that the microfilaments are removably attached to the pin.

14. The proto microfilament assembly according to claim 1, wherein the transient attachment of the microfilament guiding structure (1, 101) to the hollow guiding member is established by threads comprised in the microfilament guiding structure and attached at a proximal region to the elongated hollow guiding member.

15. The proto microfilament assembly according to claim 13 or 14, further comprising a hollow supporting guide (102) between the microfilament guiding structure (101) and the pin (4) and disposed within the elongated hollow guiding member, the hollow supporting guide comprising a central conduit (102a) accommodating the microfilaments, the pin being disposed inside the elongated hollow guiding member and proximal with respect to the hollow supporting guide to provide a gap (501a) between the hollow supporting guide and the pin, the structural rigidity of the microfilaments preferably enhanced along a section between the hollow supporting guide (102) and the distal end of the pin such as to prohibiting or essentially prohibiting individual microfilaments to bend or buckle when the pin is moved in distal direction.

16. The proto microfilament assembly according to claim 1, further comprising a pin guiding structure (3), wherein the pin is transiently attached to the pin guiding structure, and the pin guiding structure configured to be slidably disposed inside the elongated hollow guiding member.

17. The proto microfilament assembly according to any one of the preceding claims, wherein the pin comprises a recess laterally along at least a section of the pin, preferably from the distal end to a location between the distal and proximal end, optionally along the entire length of the pin, the recess configured for the accommodation of the microfilaments, the microfilament preferably bundled with a glue.

18. The proto microfilament assembly according to claim 17, wherein the microfilaments are not disposed inside the entire axial length of the pin, the microfilaments deviating laterally from the pin and the elongated hollow guiding member.
